# EUROPÄISCHE PATENTSCHRIFT

(11) **EP 3 243 456 B1**
(45) Veröffentlichungstag und Bekanntmachung des Hinweises auf die Patenterteilung: **19.02.2020**
(21) Anmeldenummer: 17159784.2
(22) Anmeldetag: 08.03.2017
(51) Int. Cl.: A61B 17/88, A61C 5/64, B05C 17/005, B65D 81/32

(54) **LAGERUNGS- UND MISCHSYSTEM FÜR PASTENFÖRMIGE AUSGANGSKOMPONENTEN MIT AUSPRESSBARER INNENKARTUSCHE**
STORAGE AND MIXING SYSTEM FOR PASTY INITIAL COMPONENTS WITH SQUEEZABLE INTERNAL CARTRIDGE
SYSTÈME DE STOCKAGE ET DE MÉLANGE POUR COMPOSANTS DE SORTIE PÂTEUX À L'AIDE D'UNE CARTOUCHE INTÉRIEURE COMPRESSIBLE

(30) Priorität: 28.04.2016 DE 102016107911
(43) Veröffentlichungstag der Anmeldung: 15.11.2017
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Sterzel, Roland

(56) Entgegenhaltungen:
- EP-A2- 2 596 873
- DE-U1-202014 102 416
- US-A- 4 691 845
- US-A- 5 405 056

## Beschreibung

Die Erfindung betrifft ein Lagerungs- und Mischsystem für pastenförmige Mehrkomponenten-Polymethylmethacrylat-Knochenzemente, das Lagerungs- und Mischsystem aufweisend eine erste röhrenförmige Kartusche mit einem Innenraum.

Die Erfindung betrifft auch ein Verfahren zur Vermischung von pastenförmigen Ausgangskomponenten eines Knochenzements, insbesondere eines pastenförmigen Mehrkomponenten-Polymethylmethacrylat-Knochenzements, mit einem solchen Lagerungs- und Mischsystem.

Gegenstand der Erfindung ist somit ein einfaches, kostengünstig herzustellendes Lagerungs- und Mischsystem für pastenförmige Mehrkomponenten-Polymethylmethacrylat-Knochenzemente, mit dem hochviskose, pastenförmige Ausgangskomponenten des Polymethylmethacrylat-Knochenzements auch mit manuell bedienbaren Auspressvorrichtungen gemischt und ausgetragen werden können.

Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Diese Polymethylmethacrylat-Knochenzemente werden nach Vermischung des Zementpulvers mit der flüssigen Monomerkomponente im noch nicht ausgehärteten, pastenförmigen Zustand als Zementteig appliziert. Bei Verwendung von Mischsystemen befindet sich der Zementteig im Fall von Pulver-Flüssigkeits-Zementen in einer Kartusche. Aus dieser Kartusche wird der Zementteig durch Bewegung eines Austragskolbens heraus gedrückt. Die Austragskolben haben üblicherweise einen Durchmesser zwischen 30 mm bis 40 mm und damit an der Außenseite, an welcher der Stößel der Auspressvorrichtung beim Auspressvorgang angreift, eine Fläche von 7,0 cm² bis 12,5 cm². Die Bewegung des Austragskolbens wird durch manuell bedienbare, mechanische Auspressvorrichtungen bewirkt, die auch als Applikatoren bezeichnet werden. Diese Auspressvorrichtungen beziehungsweise Applikatoren haben normalerweise eine Auspresskraft im Bereich von ungefähr 1,5 kN bis 3,5 N.

Eine neuere Entwicklung stellen pastenförmige Zweikomponenten-Knochenzemente dar, wie sie beispielsweise aus der DE 10 2007 050 762 B3, der DE 10 2008 030 312 A1 und der DE 10 2007 052 116 B4 bekannt sind. Bei diesen Zweikomponenten-Knochenzementen werden zwei pastenförmige Ausgangskomponenten in zwei separaten Kartuschen mit zwei separaten Austragskolben aufbewahrt. Bei der Applikation werden beide Pasten durch Bewegung der Austragkolben aus den Kartuschen in einen statischen Mischer gepresst und nach erfolgter Vermischung durch ein Austragsrohr ausgetragen. Bei geeigneter Zusammensetzung der pastenförmigen Ausgangskomponenten wird nach der Vermischung der beiden Ausgangskomponenten sofort ein klebfreier, applikationsbereiter Zementteig erhalten. Wartezeiten bis zum Erreichen der Klebfreiheit des Zementteigs, die bei bisherigen konventionellen Polymethylmethacrylat-Knochenzementen immer zwangsläufig auftreten, entfallen dadurch. Wertvolle OP-Zeit kann dadurch gespart werden.

Bei der Applikation der bisherigen, konventionellen PMMA-Knochenzemente, die aus einer flüssigen Monomerkomponente und einer separat dazu gelagerten Zementpulverkomponente als Ausgangskomponenten bestehen, wird nach der Vermischung der beiden Ausgangskomponenten in Zementiersystemen beziehungsweise Vakuumzementiersystemen der gebildete Zementteig mit Hilfe von manuell bedienbaren Auspressvorrichtungen ausgepresst. Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei hochviskosen pastenförmigen Ausgangskomponenten unter Verwendung von Kartuschen, bei denen die Austragskolben an der äußeren Kolbenseiten, an denen die Stößel der Auspressvorrichtungen angreifen, eine gesamte Fläche im Bereich von 7,0 cm² bis 12,5 cm² haben, sind diese Vorrichtungen nicht oder nur mit einem sehr großen Kraftaufwand manuell zu bedienen. Dieser große Kraftaufwand ist medizinischen Anwendern im OP nicht zuzumuten.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A; DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1; US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

Die US 8 544 683 B2 offenbart ein Kartuschensystem, das zur Beimischen einer geringen Menge zu einer Hauptausgangskomponente geeignet ist. Bei dem Kartuschensystem ist neben einer Kartusche eine zweite kleinere Kartusche angeordnet, wobei bei einem Vortrieb eines Austragskolbens in der größeren Kartusche auch ein Austragskolben in der kleineren Kartusche über ein gemeinsames Verbindungselement angetrieben wird. Das System ist zum Mischen der zähen pastösen Ausgangskomponenten von PMMA-Knochenzement jedoch nicht geeignet.

Die DE 20 2014 102 416 U1 offenbart eine Zweikomponenten-Klebepatrone, bei der eine Hülse mit einer Trennscheibe in zwei Bereiche getrennt wird. Beim Auspressen des Inhalts mit zwei Schiebern wird die Trennscheibe mit zwei Schneiden hinter den Schiebern aufgeschnitten. Aus der US 5 405 056 A ist ein Austragsbehälter mit einem innenliegenden Blister bekannt, wobei der Blister mit einem Kolben ausgequetscht wird.

Ein koaxiales Kartuschensystem, das ein spezielles Kolbensystem enthält, wird im Patent EP1 392 450 B1 beschrieben. Das Kartuschensystem findet Anwendung in der Baustoffchemie für die Lagerung und Vermischung von pastenförmigen Zweikomponentendichtungsmassen. Das dort offenbarte Kolbensystem hat einen zylinderförmigen Austragskolben für die zentrale Kartusche und einen ringförmigen Austragskolben für die zweite, koaxial angeordnete Kartusche. Beide Austragskolben werden hinter den Dichtungsflächen über ein Auflageelement angetrieben, das auf der Rückseite mehrere Anlageflächen für den Stößel der Auspressvorrichtung besitzt. Das Auflageelement enthält bogenförmige Schneiden. Bei axialer Einwirkung des Stößels einer Auspressvorrichtung werden beide Austragskolben vorwärts in Richtung Kartuschenkopf bewegt. Dabei werden die in den koaxialen Kartuschen enthaltenen pastenförmigen Komponenten in Richtung Kartuschenkopf gedrückt. Gleichzeitig zerteilen zwei Schneiden die Wand der inneren koaxialen Kartusche in zwei Teile. Nachteilig ist an diesem System, dass zwangsläufig gleichzeitig zwei Schneidprozesse ablaufen. Das bedeutet, dass für beide Schneidprozesse Energie aufgebracht werden muss, die dadurch nicht zum eigentlichen Vortrieb der beiden pastöse Komponenten zur Verfügung steht. Die Vermischung von pastenförmigen Ausgangskomponenten für PMMA-Knochenzemente benötigt aufgrund der im Austragsrohr angeordneten statischen Mischer und der hohen Viskosität der Ausgangskomponenten sehr viel Vortriebsenergie, der bei größeren Kartuschen nicht mehr manuell, gefahrlos und nicht mit herkömmlichen Auspressvorrichtungen aufzubringen ist. Ein Verlust an Vortriebsenergie durch zwei gleichzeitig stattfindende Schneidprozesse kann daher insbesondere bei hoch viskosen pastenförmigen Komponenten problematisch sein. Zudem sind Koaxialkartuschen nicht ohne weiteres mit der zähen pastenförmigen Haupt-Ausgangskomponente eines PMMA-Knochenzements zu befüllen. Insbesondere wenn nur geringe Mengen des PMMA-Knochenzements enthalten sein sollen, werden die freien Querschnitte der äußeren Koaxialkartusche für die Hauptausgangskomponente so klein, dass sie nicht mit herkömmlichen Verfahren zu befüllen ist.

Im Patent FR 1 468 507 wird ein Kartuschensystem offenbart, bei dem in einer Kartusche ein schlauchförmiger Lagerbehälter angeordnet ist. Dieser ist am Kartuschenende an einem Punkt mit der Kartusche verbunden. In der Kartusche ist ein Austragskolben angeordnet, der eine Öffnung besitzt, in der ein Teil des schlauchförmigen Lagerbehälters angeordnet ist, wobei die Öffnung kleiner ist als der Durchmesser des schlauchförmigen Lagerbehälters. Bei der Vorwärtsbewegung des Austragskolbens in Richtung Kartuschenkopf wird einerseits die in der Kartusche enthaltene Masse ausgepresst und andererseits wird aus dem schlauchförmigen Behälter die darin enthaltene Masse durch Ausquetschen in Richtung Kartuschenkopf bewegt. Für die Funktion des Ausquetschens ist es wichtig, dass der schlauchförmige Lagerbehälter am Ende der Kartusche fixiert ist, damit beim Ausquetschen der schlauchförmige Lagerbehälter sich nicht mit dem Austragskolben nach vorne in Richtung Kartuschenkopf mit bewegt, ohne dass die darin enthaltene Masse ausgedrückt wird. Nachteilig ist an dem vorgeschlagenen System, dass durch das einfache Ausquetschen an einer Öffnung zwangsläufig mehr oder weniger große Reste der auszupressenden Masse im schlauchförmigen Lagerbehälter verbleiben. Beim Ausquetschen wirft der Schlauch unkontrollierbar und unvorhersehbare Falten, in denen Reste des auszuquetschenden Materials zurückbleiben. Dadurch ist die Verwendung dieses Lagerungssystems bei Mehrkomponenten-Knochenzementpasten nicht oder nur bedingt möglich, weil für die reproduzierbare Aushärtung des Knochenzements exakt definierte Gehalte der mindestens zwei Initiatorkomponenten erforderlich sind, die getrennt in der ersten Ausgangskomponente und in der zweiten Ausgangskomponente angeordnet sind. Eine Variation des Mischungsverhältnisses muss daher immer soweit als möglich vermieden werden. Zudem müssten die Kartuschen mit den Resten der Ausgangskomponenten aufgrund der chemischen Zusammensetzung der Ausgangskomponenten aufwendig entsorgt werden.

Die Aufgabe der Erfindung besteht also darin, die Nachteile des Stands der Technik zu überwinden. Insbesondere soll ein einfaches, kostengünstig zu fertigendes Lagerungs- und Mischsystem für pastenförmige Mehrkomponenten-Polymethylmethacrylat-Knochenzemente und ein Verfahren zur Herstellung eines Zementteigs mit einem Lagerungs- und Mischsystem bereitgestellt werden, wobei das Lagerungs- und Mischsystem als einmalig zu verwendendes ready-to-use-System in einfachster Weise mit einer minimalen Anzahl von Montageschritten innerhalb weniger Sekunden einsatzbereit machbar sein soll und nach Verbindung mit manuell anzutreibenden medizinischen Auspressvorrichtungen beziehungsweise Applikatoren sofort nach Beginn der manueller Betätigung der Auspressvorrichtung einen homogen gemischten Zementteig erzeugt und an der Austragsöffnung eines Austragsrohrs austrägt. Die in den OPs bisher für die konventionellen Polymethylmethacrylat-Knochenzemente genutzten manuell zu bedienenden Auspressvorrichtungen mit jeweils einer Schubstange beziehungsweise mit jeweils einem Stößel und gegebenenfalls einem Teller sollen für den Austrag des Zweikomponenten-Polymethylmethacrylat-Knochenzements beziehungsweise des Zementteigs mit dem zu entwickelnden Lagerungs- und Mischsystem genutzt werden können. Dadurch soll die Beschaffung von speziellen Auspressvorrichtungen für den Austrag von pastenförmigen Zweikomponenten-Polymethylmethacrylat-Knochenzementen vermieden werden.

Bevorzugt soll das zu entwickelnde Lagerungs- und Mischsystem keine zwei miteinander verbundenen synchron vorzutreibende Schubstangen beziehungsweise Stößel erfordern, damit die gesamte Vorrichtung nicht wesentlich länger und größer wird als die bisher für die konventionellen Pulver-Flüssigkeits-Polymethylmethacrylat-Knochenzemente üblichen Mischsysteme und Vakuummischsysteme. Es soll eine einfache Lösung gefunden werden, die es erlaubt, möglichst mit nur einer Schubstange beziehungsweise nur einem Stößel und gegebenenfalls einem daran befestigten Teller zwei oder mehr pastenförmige Ausgangskomponenten synchron und manuell aus der Vorrichtung auszutreiben. Die pastenförmigen Ausgangskomponenten des Knochenzements sollen getrennt innerhalb des Lagerungs- und Mischsystems sicher gelagert werden können. Zur Anwendung sollen beide pastenförmigen Ausgangskomponenten sicher zusammengeführt werden können.

Es soll auch möglich sein, dass die beiden Ausgangskomponenten quantitativ aus den Kartuschen ausgepresst werden können, damit das Mischungsverhältnis der Initiatorkomponenten reproduziert werden kann, um damit reproduzierbare Verarbeitungseigenschaften des gemischten Zementteigs und der mechanischen Eigenschaften des ausgehärteten Zements zu gewährleisten.

Das Lagerungs- und Mischsystem soll auch ein geringes Volumen des homogen gemischten Zementteigs von ca. 40 ml beziehungsweise maximal 70 ml austragen können, ohne dass größere Restmengen (mehr als 15 ml) in dem System verbleiben und aufwendig entsorgt werden müssen. Größere Volumina des Zementteigs werden für bevorzugte Anwendungen nicht angestrebt. Die genannten geringen Mengen sind nämlich für viele solcher Anwendungen ausreichend, wie beispielsweise Operationen (OPs) im Bereich des Knies.

Die Lagerungs- und Mischvorrichtung soll es erlauben, zwei Pasten im Volumenverhältnis größer oder gleich 95 zu 5 oder bevorzugt größer oder gleich 98 zu 2 zu lagern und auszupressen. Die Vorrichtung ist dabei für solche pastenförmige Mehrkomponenten-Polymethylmethacrylat-Knochenzemente vorgesehen, bei denen die sich eine geringvolumige pastenförmige Ausgangskomponente sehr leicht mit einer großvolumigen pastenförmige Ausgangskomponente vermischen lässt, wobei sich die geringvolumige pastenförmige Ausgangskomponente in der großvolumigen pastenförmige Ausgangskomponente innerhalb von wenigen Sekunden löst.

Der Übergang von der Kartusche zum Austragsrohr soll konstruktiv möglichst so gestaltet sein, dass der Strömungswiderstand der pastenförmigen Ausgangskomponenten beim Auspressen möglichst niedrig ist. Als Ausgangskomponenten sollen bevorzugt pastenförmige Ausgangskomponenten verwendet werden, die unmittelbar nach dem Auspressen anwendbar sind, bei denen also keine Zeit zum Anquellen des PMMA-Knochenzements notwendig ist. Die Vorrichtung soll so beschaffen sein, dass eine Verwechselung der relevanten Montageschritte durch den Anwender konstruktiv soweit wie möglich ausgeschlossen ist und das Lagerungs- und Mischsystem auch von weitgehend ungeschultem Personal durchführbar ist. Weiterhin soll ein Verfahren zum Vermischen der pastenförmigen Ausgangskomponenten und zum Austragen des homogen gemischten Zementteigs bereitgestellt werden.

Die Aufgaben der Erfindung werden gelöst durch ein Lagerungs- und Mischsystem für pastenförmige Mehrkomponenten-Polymethylmethacrylat-Knochenzemente, das Lagerungs- und Mischsystem aufweisend
a) eine erste röhrenförmige Kartusche mit einem ersten zylindrischen Innenraum, wobei in dem Innenraum eine erste Ausgangskomponente eines Mehrkomponenten-Knochenzements enthalten ist,
b) einen ersten Austragskolben der axial beweglich im ersten Innenraum der ersten Kartusche angeordnet ist und der zum Austreiben der ersten Ausgangskomponente aus der ersten Kartusche durch eine dem ersten Austragskolben gegenüberliegende Öffnung in einem Kartuschenkopf der ersten Kartusche vorgesehen ist,
c) eine zweite röhrenförmige Kartusche, die innerhalb der ersten röhrenförmigen Kartusche angeordnet ist, wobei die zweite Kartusche mit ihrer Außenwand an der Innenwand der ersten Kartusche anliegt und an der Innenwand der ersten Kartusche befestigt ist, wobei in der zweiten Kartusche eine zweite Ausgangskomponente des Mehrkomponenten-Knochenzements enthalten ist und ein zweiter Austragskolben angeordnet ist, wobei mit dem zweiten Austragskolben die zweite Ausgangskomponente aus der zweiten Kartusche durch eine gegenüberliegende Öffnung in der zweiten Kartusche im Bereich des Kartuschenkopfs der ersten Kartusche auszutreiben ist,
d) wobei vom Kartuschenkopf aus gesehen hinter dem ersten Austragskolben und dem zweiten Austragskolben eine axial im Innenraum der ersten Kartusche vortreibbare Pressvorrichtung mit einer Klemmkante zum Zusammenpressen der zweiten Kartusche angeordnet ist, wobei die Pressvorrichtung derart in Richtung des Kartuschenkopfs vortreibbar ist, dass während der Bewegung der Pressvorrichtung die zweite Kartusche axial fortlaufend zusammengepresst wird und dabei der erste Austragskolben und der zweite Austragskolben in Richtung des Kartuschenkopfs vorgetrieben werden.

Der Innenraum der ersten (äußeren) Kartusche hat vorzugsweise eine zylindrische Geometrie. Das gilt auch für den Innenraum der zweiten (inneren) Kartusche vor der Verformung der Wand der zweiten Kartusche. Die zylindrische Form ist die einfachste, mit der sich die Innenräume der Kartuschen realisieren lassen. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die begrenzende Innenwand des Innenraums kann also ein Zylinder mit beliebiger Grundfläche sein und der Mantel kann ein Zylinder mit beliebiger Grundfläche sein, das heißt auch mit nicht kreisförmiger oder runder Grundfläche. Erfindungsgemäß wird jedoch eine zylindrische Geometrie mit rotationssymmetrischer und insbesondere kreisrunder Grundfläche für den Innenraum der ersten Kartusche bevorzugt, da diese am einfachsten zu fertigen sind. Die Wand der zweiten Kartusche kann derart an der Innenwand der ersten Kartusche befestigt sein, dass eine zylindrische Symmetrie des zweiten Innenraums von der kreisförmigen Grundfläche abweicht.

Die Pressvorrichtung und der erste Austragskolben können auch einteilig oder fest miteinander verbunden ausgeführt sein.

Die Ausgangskomponenten des Mehrkomponenten-Knochenzements, insbesondere des Mehrkomponenten-Polymethylmethacrylat-Knochenzements, sind bevorzugt fluid, besonders bevorzugt pastenförmig.

Der erste Austragskolben schließt bevorzugt dicht mit der Innenwand des ersten zylindrischen Innenraums ab und der zweite Austragskolben schließt dicht mit der Innenwand des zweiten zylindrischen Innenraums ab. Der zweite zylindrische Innenraum ist dabei nicht verformt. Besonders bevorzugt schließt der erste Austragskolben dicht mit der Außenwand der zweiten Kartusche in dem Bereich ab, in denen die Außenwand der zweiten Kartusche den Innenraum der ersten Kartusche begrenzt.

Mit der vorliegenden Erfindung wird vorgeschlagen, dass die zweite Kartusche mit ihrer Außenwand mit der Innenwand der ersten Kartusche vorne im Bereich des Kartuschenkopfs und hinten, hinter dem zweiten Austragskolben befestigt ist, wobei vorzugsweise die zweite Kartusche mit ihrer Außenwand mit der Innenwand der ersten Kartusche entlang der gesamten Länge der zweiten Kartusche befestigt ist.

Hierdurch wird verhindert, dass sich die zweite Kartusche unkontrolliert in der ersten Kartusche bewegt und dadurch eine Undichtigkeit der sich verformenden zweiten Kartusche entsteht. Eine Verbindung beziehungsweise Befestigung der zweiten Kartusche entlang der gesamten Länge der zweiten Kartusche ist dazu besonders gut geeignet. Zudem kann dies auch einfach gefertigt werden.

Es kann erfindungsgemäß vorgesehen sein, dass die Öffnungen mit einem lösbaren Verschluss, insbesondere mit lösbaren Stopfen, an dem Kartuschenkopf verschlossen sind.

Hiermit werden die Innenräume des Lagerungs- und Mischsystems seitlich durch die zylindrischen Innenräume, rückseitig durch die Austragskolben und vorderseitig durch die lösbaren Verschlüsse nach außen abgeschlossen. Dadurch ist das Lagerungs- und Mischsystem zur langfristigen Lagerung der Ausgangskomponenten geeignet.

Es kann auch vorgesehen sein, dass beim Vortreiben der Pressvorrichtung der erste Austragskolben und der zweite Austragskolben parallel zueinander vorgetrieben werden, vorzugsweise der erste Austragskolben und der zweite Austragskolben auf gleicher Höhe in Richtung des Kartuschenkopfs laufen.

Hiermit wird eine gleichmäßige Mischung der Ausgangskomponenten erreicht, bei der das Mischungsverhältnis gleichbleibend ist und damit die Eigenschaften des zu mischenden Knochenzements.

Des Weiteren kann vorgesehen sein, dass im Bereich des Kartuschenkopfs außen an der ersten Kartusche ein Befestigungsmittel, insbesondere ein Außengewinde, vorgesehen ist.

Hiermit kann einerseits ein Verschluss zum Verschließen der Öffnungen der Kartuschen an der ersten Kartusche befestigt werden und andererseits ein Austragsrohr und ein statischer Mischer an der Vorderseite der Öffnungen befestigt werden. Diese müssen ein passendes Gegenbefestigungsmittel aufweisen, bevorzugt ein passendes Innengewinde.

Bei bevorzugten Lagerungs- und Mischsystemen kann vorgesehen sein, dass das Lagerungs- und Mischsystem ein Austragsrohr mit einem statischen Mischer aufweist, das an der ersten Kartusche zu befestigen ist, vorzugsweise an dem Befestigungsmittel an der ersten Kartusche, zu befestigen ist, wobei besonders bevorzugt an dem Austragsrohr ein zum Außengewinde an der ersten Kartusche passendes Innengewinde vorgesehen ist und/oder Elemente eines Bajonettverschlusses und/oder Rastelemente eines Rastverschlusses vorgesehen sind.

Hiermit wird das Lagerungs- und Mischsystem weiter vervollständigt. Das Lagerungs- und Mischsystem ist dann auch zur Applikation des gemischten Knochenzements geeignet. Mit dem statischen Mischer wird eine stärkere Durchmischung der Ausgangskomponenten erreicht und damit ein homogenerer Knochenzementteig erzeugt.

Dabei kann vorgesehen sein, dass das Verhältnis vom Innendurchmesser der ersten Kartusche zum Innendurchmesser des Austragsrohrs kleiner 5 zu 2 ist, wobei bevorzugt das Verhältnis vom Innendurchmesser der ersten Kartusche zum Innendurchmesser des Austragsrohr bevorzugt kleiner oder gleich 2 zu 1 ist und ganz besonders bevorzugt das Verhältnis vom Innendurchmesser der ersten Kartusche zum Innendurchmesser des Austragsrohrs 8 zu 5 ist.

Hierdurch wird erreicht, dass während des Vortreibens der Austragskolben eine ausreichende Strömungsgeschwindigkeit des Knochenzements an der Austragsöffnung des Austragsrohrs erzeugt wird.

Es kann auch vorgesehen sein, dass der Innendurchmesser der ersten Kartusche höchstens 25 mm ist und der Innendurchmesser des Austragsrohrs höchstens 15 mm ist, wobei bevorzugt der Innendurchmesser der Kartusche höchstens 20 mm ist und der Innendurchmesser des Austragsrohrs höchstens 12 mm ist.

Durch den erfindungsgemäßen Aufbau der Kartuschen beziehungsweise der Kartuschen und des Austragsrohrs gelingt es, die oft besonders zähen und pastösen Ausgangskomponenten des Knochenzements, insbesondere bezüglich der ersten Ausgangskomponente, in einer einzigen Kartusche unterzubringen, die noch durch manuelle Krafteinwirkung auspressbar ist und die aber auch gleichzeitig noch mit herkömmlichen Techniken befüllt werden können. Bei größeren Durchmessern reicht eine manuelle Krafteinwirkung nicht mehr ohne weiteres aus, um die zähflüssigen pastösen Ausgangskomponenten des Knochenzements aus der Kartusche zu pressen. Bei den angegebenen Durchmessern wirken sich die Vorteile der vorliegenden Erfindung also besonders stark aus.

Ferner kann vorgesehen sein, dass das Verhältnis des Innendurchmessers der ersten Kartusche zum Abstand zwischen dem ersten Austragskolben und dem Kartuschenkopf höchstens 1 zu 10 ist, wobei bevorzugt das Verhältnis des Innendurchmessers der ersten Kartusche zum Abstand zwischen dem ersten Austragskolben und dem Kartuschenkopf höchstens 1 zu 15 ist.

Damit wird sichergestellt, dass die Verformung der zweiten Kartusche die Bewegung der Pressvorrichtung nicht zu stark behindert, so dass das Lagerungs- und Mischsystem noch mit vertretbarer manueller Krafteinwirkung angetrieben werden kann.

Bevorzugte Lagerungs- und Mischsysteme können sich auch dadurch auszeichnen, dass die Klemmkante gegen die Längsachse der ersten Kartusche geneigt ist, vorzugsweise mit einem Winkel von wenigstens 40° senkrecht zur Längsachse in Richtung der Innenwand der ersten Kartusche geneigt ist, besonders bevorzugt mit einem Winkel zwischen 40° und 80° senkrecht zur Längsachse in Richtung der Innenwand der ersten Kartusche geneigt ist.

Hierdurch wird die Wand der zweiten Kartusche über eine größere geneigte Fläche verformt, wodurch der Vortrieb vereinfacht wird und die Verformung gleichmäßiger erfolgen kann. Gleichzeitig ist die Verformung aber ausreichend, um den zweiten Austragskolben rückseitig durch die sich verformende Wand der zweiten Kartusche gleichmäßig anzutreiben.

Mit der Erfindung wird auch vorgeschlagen, dass die Klemmkante beim Vortreiben der Pressvorrichtung die zweite Kartusche beziehungsweise die Wand der zweiten Kartusche gegen die Innenwand der ersten Kartusche quetscht.

Damit wird die Wand der Kartusche so weit als möglich aus dem Wirkungsbereich eines nachfolgenden Stößels einer Auspressvorrichtung gedrückt, so dass diese nicht dessen Bewegung behindern kann.

Des Weiteren kann vorgesehen sein, dass die Klemmkante mindestens 30 Prozent der Fläche des Querschnitts der zweiten Kartusche überdeckt, bevorzugt mindestens 60% der Fläche des Querschnitts der zweiten Kartusche überdeckt.

Hiermit wird eine ausreichende Umformung erreicht, damit der zweite Austragskolben durch die sich verformende Wand der zweiten Kartusche angetrieben werden kann. Ferner wird so auch die Wand der Kartusche ausreichend weit aus dem Wirkungsbereich eines nachfolgenden Stößels einer Auspressvorrichtung gedrückt, so dass diese nicht dessen Bewegung behindern kann.

Bevorzugt kann auch vorgesehen sein, dass zwischen der Pressvorrichtung und der Innenwand der ersten Kartusche im Bereich der zweiten Kartusche ein Spalt vorgesehen ist, der genauso breit oder breiter ist als die Dicke der Wand der zweiten Kartusche.

Hierdurch kann die Pressvorrichtung über die verformte zweite Kartusche laufen, ohne dass das Material der Wand der zweiten Kartusche in sich komprimiert werden müsste und ohne dass eine Verformung der ersten Kartusche notwendig wäre, was zu einem unerwünschten zusätzlichen Kraftaufwand beim Vortreiben der Pressvorrichtung führen würde.

Es wird ferner vorgeschlagen, dass die Rückseite der Pressvorrichtung als Auflagefläche für einen Stößel einer Auspressvorrichtung ausgebildet ist.

Hiermit kann die Pressvorrichtung leicht mit einer herkömmlichen Auspressvorrichtung angetrieben werden.

Gemäß einer bevorzugten Weiterbildung kann vorgesehen sein, dass der Durchmesser des Innenraums der ersten Kartusche kleiner oder gleich 25 mm ist, wobei bevorzugt der Durchmesser des Innenraums der ersten Kartusche kleiner oder gleich 20 mm ist, und/oder die erste Kartusche einen Innendurchmesser von höchstens 25 mm und die zweite Kartusche einen Innendurchmesser von höchstens 5 mm haben, bevorzugt die erste Kartusche einen Innendurchmesser von höchstens 20 mm und die zweite Kartusche einen Innendurchmesser von höchstens 3 mm haben.

Durch den erfindungsgemäßen Aufbau der ersten Kartuschen beziehungsweise der ersten und der zweiten Kartusche gelingt es, die oft besonders zähen und pastösen Ausgangskomponenten des Knochenzements, insbesondere bezüglich der ersten Ausgangskomponente, in einem einzigen Lagerungs- und Mischsystem unterzubringen, das noch durch manuelle Krafteinwirkung auspressbar ist und die aber auch gleichzeitig noch mit herkömmlichen Techniken befüllt werden kann. Bei größeren Durchmessern reicht eine manuelle Krafteinwirkung nicht mehr ohne weiteres aus, um die zähflüssigen pastösen Ausgangskomponenten des Knochenzements aus der Kartusche beziehungsweise den Kartuschen zu pressen. Bei den angegebenen Durchmessern wirken sich die Vorteile der vorliegenden Erfindung also besonders stark aus.

Es kann auch vorgesehen sein, dass beim Vortreiben der Pressvorrichtung in Richtung Kartuschenkopf die Klemmkante durch Quetschung der zweiten Kartusche die so deformierte Wand der zweiten Kartusche gegen die Unterseite des zweiten Austragskolbens gepresst wird und so den zweiten Austragskolben in Richtung Kartuschenkopf schiebt.

Hiermit kann ein separater oder komplexer Antrieb für den zweiten Austragskolben vermieden werden. Ohne den zweiten Austragskolben käme es aber bei einem reinen Ausquetschen der zweiten Ausgangskomponente aus der sich verformenden zweiten Kartusche zu ungewollten Variationen in der Zusammensetzung des Knochenzements, da sich beim Verformen der zweiten Kartusche unkontrolliert Falten bilden, in denen unvorherbestimmbare Reste der zweiten Ausgangskomponente verbleiben, die nicht mit der ersten Ausgangskomponente gemischt werden. Dagegen wird beim Vortreiben des zweiten Austragskolbens, der mit Dichtungen und/oder Abstreiflippen gegen die Innenwand der zweiten Kartusche abgedichtet werden kann, immer der gesamte Inhalt der zweiten Kartusche gefördert und ausgetrieben.

Bevorzugte Ausführungsformen der Erfindung können sich dadurch auszeichnen, dass die erste Kartusche, die zweite Kartusche, der Kartuschenkopf und die Austragskolben aus Kunststoff gefertigt sind, wobei als Kunststoffe Polyethylen-covinylalkohol (EVOH), Polybutylenterephthalat (PBT), Polyethylenterephthalat (PET) und Poly-methacrylsäuremethylester-co-acrylnitril bevorzugt werden.

Der Aufbau mit Kunststoffen ist kostengünstig und einfach umsetzbar. Die bevorzugten Kunststoffe sind aufgrund ihrer Resistenz gegenüber den in den Ausgangskomponenten enthaltenen Chemikalien besonders gut geeignet.

Es wird mit der vorliegenden Erfindung vorgeschlagen, dass die Pressvorrichtung oder die Klemmkante aus Metall und/oder Kunststoff und/oder glasfaserverstärktem Kunststoff gefertigt ist, wobei die Pressvorrichtung oder die Klemmkante aus Stahl, Aluminiumlegierungen, Zinklegierungen, Polyamid, glasfaserverstärktem Polyamid, Polyetherketon, Polysulfon oder Kombinationen dieser Werkstoffe gefertigt ist.

Durch die Härte dieser Materialien kann eine ausreichende Umformung der zweiten Kartusche erreicht werden, ohne dass sich die Klemmkante selbst zu stark verformt.

Bevorzugte Lagerungs- und Mischsysteme können sich auch dadurch auszeichnen, dass das Verhältnis des Volumens der ersten Kartusche zum Volumen der zweiten Kartusche mindestens 95 zu 5 ist, bevorzugt mindestens 98 zu 2 ist.

Hierdurch werden kleine beziehungsweise geringe Mengen einer zweiten Komponente beigemischt und so die Vorteile des erfindungsgemäßen Aufbaus besonders gut genutzt. Das erfindungsgemäße Lagerungs- und Mischsystem zeichnet sich nämlich dadurch aus, dass auch solche stark unterschiedlichen Mischungsverhältnisse noch homogen erzeugt werden können.

Des Weiteren kann vorgesehen sein, dass die zweite Kartusche bei einer Beaufschlagung der Pressvorrichtung von mindestens 0,5 kN in Richtung des Kartuschenkopfs so gequetscht wird, dass die gequetschte zweite Kartusche durch einen Spalt zwischen der Klemmkante und der Innenwand der ersten Kartusche passt.

Bevorzugt wird dies durch die Wahl einer geeigneten Dicke der Wand der zweiten Kartusche erreicht sowie durch die Auswahl eines geeigneten Materials für die zweite Kartusche. Hiermit wird erreicht, dass das Lagerungs- und Mischsystem mit manuellem Kraftaufwand auspressbar beziehungsweise verwendbar ist.

Ferner kann vorgesehen sein, dass das Verhältnis der Dicke der Wand der ersten Kartusche zu der Dicke der Wand der zweiten Kartusche mindestens 11 zu 10 ist, und wobei bevorzugt das Verhältnis der Dicke der Wand der ersten Kartusche zu der Dicke der Wand der zweiten Kartusche mindestens 2 zu 1 ist, besonders bevorzugt mindestens 3 zu 1 ist.

Dies gilt für den Fall, dass die erste und die zweite Kartusche aus dem gleichen Material bestehen. Sofern die innere zweite Kartusche aus einem Material mit einem geringeren Elastizitäts-Modul beziehungsweise einem leichter umformbaren Material besteht, kann die Wandstärke der zweiten inneren Kartusche auch gleich groß oder sogar größer gewählt werden, als die Wandstärke der äußeren Kartusche. Hiermit wird erreicht, dass sich die innere zweite Kartusche verformen lässt, ohne, dass sich die äußere erste Kartusche mit verformt und dadurch die Bewegung der Pressvorrichtung und der Austragskolben behindert wird.

Die innere zweite Kartusche kann als fertiges Teil in die erste Kartusche eingeschweißt werden. Dies ist insbesondere dann wichtig, wenn die beiden Kartuschen nicht aus dem gleichem Material bestehen.

Mit einer Weiterbildung der vorliegenden Erfindung wird vorgeschlagen, dass ein Teil der Wand der ersten Kartusche einen Teil der Innenwand der zweiten Kartusche bildet, bevorzugt über die gesamte Länge der zweiten Kartusche oder über zumindest 80% der gesamten Länge der zweiten Kartusche, und/oder dass ein Teil der Wand der ersten Kartusche einen Teil des zweiten Innenraums des zweiten Kartusche begrenzt, bevorzugt über die gesamte Länge der zweiten Kartusche oder über zumindest 80% der gesamten Länge der zweiten Kartusche.

Hiermit ist eine Bewegung der zweiten Kartusche gegen die erste Kartusche besonders effektiv eingeschränkt. Zudem kann so die Verformung der Wand der zweiten Kartusche besonders gut vorgegeben werden.

Es kann vorgesehen sein, dass die Dicke der Wand der ersten Kartusche mindestens 1 mm ist und die Wand der zweiten Kartusche höchstens 0,5 mm ist.

Hiermit wird erreicht, dass sich nur die zweite Kartusche plastisch verformt, während die erste Kartusche plastisch unverformt bleibt.

Es kann erfindungsgemäß vorgesehen sein, dass die zweite Kartusche von der Klemmkante plastisch verformt wird, beziehungsweise plastisch verformbar ist.

Es kann erfindungsgemäß ferner vorgesehen sein, dass die erste Kartusche an der dem Kartuschenkopf gegenüberliegenden Seite außen an der Wand ein Befestigungsmittel aufweist, mit der das Lagerungs- und Mischsystem mit einer Auspressvorrichtung verbindbar ist.

Des Weiteren kann vorgesehen sein, dass der Kartuschenkopf mit einer gummielastischen Platte und einer aus Kunststoff gefertigten Überwurfkappe oder Überwurfmutter aufgebaut ist, wobei die Überwurfkappe oder Überwurfmutter die gummielastische Platte durch einen überragenden Rand blockiert, und wobei in der gummielastische Platte mindestens die beiden Öffnungen der ersten und zweiten Kartusche angeordnet sind, die durch einen Stopfen verschlossen sind.

Dabei kann vorgesehen sein, dass die Überwurfkappe oder Überwurfmutter als Verbindungselement ein Innengewinde oder ein Außengewinde oder einen Bajonettverschluss oder Rastelemente für einen rastenden Verschluss mit der ersten Kartusche aufweist.

Ferner kann vorgesehen sein, dass eine Kunststoffplatte mit zwei mit Stopfen verschließbare oder verschlossene Öffnungen auf oder unter der gummielastischen Platte im Kartuschenkopf angeordnet ist.

Bevorzugt kann ferner vorgesehen sein, dass die Stirnfläche der Kartusche als Kartuschenkopf ausgebildet ist, wobei mindestens zwei durch Stopfen verschließbare Öffnungen die Stirnfläche der Kartusche durchbrechen. Die verschließbaren Öffnungen bilden bevorzugt die beiden Öffnungen der ersten Kartusche und der zweiten Kartusche.

Bevorzugt kann auch vorgesehen sein, dass die Stirnfläche der Kartusche zu mindestens 40 Prozent durch eine Platte verschlossen ist, wobei in dem nicht geschlossenen Teil der Stirnfläche die Öffnung der zweiten Kartusche angeordnet ist. Dadurch wird die Vermischung der kleinvolumigen zweiten Ausgangskomponente mit der großvolumigen ersten Ausgangskomponente begünstigt.

Es kann auch vorgesehen sein, dass eine dritte röhrenförmige Kartusche innerhalb der ersten röhrenförmigen Kartusche angeordnet ist, wobei die dritte Kartusche mit ihrer Außenwand an der Innenwand der ersten Kartusche anliegt und an der Innenwand der ersten Kartusche befestigt ist, wobei in der dritten Kartusche die zweite Ausgangskomponente oder eine dritte Ausgangskomponente des Mehrkomponenten-Knochenzements enthalten ist und ein dritter Austragskolben angeordnet ist, wobei mit dem dritten Austragskolben die zweite Ausgangskomponente oder die dritte Ausgangskomponente aus der dritten Kartusche durch eine gegenüberliegende Öffnung in der dritten Kartusche im Bereich des Kartuschenkopfs der ersten Kartusche auszutreiben ist, wobei die Pressvorrichtung vom Kartuschenkopf aus gesehen hinter dem dritten Austragskolben angeordnet ist und die Pressvorrichtung eine Klemmkante zum Zusammenpressen der dritten Kartusche aufweist, wobei die Pressvorrichtung derart in Richtung des Kartuschenkopfs vortreibbar ist, dass während der Bewegung der Pressvorrichtung die dritte Kartusche axial fortlaufend zusammengepresst wird und dabei der erste Austragskolben, der zweite Austragskolben und der dritte Austragskolben in Richtung des Kartuschenkopfs vorgetrieben werden.

Hiermit kann eine weitere Ausgangskomponente in den Knochenzementteig gemischt werden. Wenn die dritte Kartusche mit der zweiten Ausgangskomponente befüllt ist, hat dies den Vorteil, dass hierdurch die zweite Ausgangskomponente an unterschiedlichen Stellen der ersten Ausgangskomponente beigemischt werden kann und so eine homogenere Mischung der Ausgangskomponenten erreicht werden kann.

Bevorzugt ist die dritte röhrenförmige Kartusche an der zur zweiten Kartusche gegenüberliegenden Innenwand der ersten Kartusche angeordnet. Durch diese Symmetrie wird erreicht, dass auch die Krafteinwirkung durch die Verformung der zweiten und dritten Kartusche symmetrisch auf die Pressvorrichtung einwirkt. Dadurch kann ein gleichmäßigerer Vortrieb erreicht werden. Insbesondere ist aber die Gefahr reduziert, dass die Pressvorrichtung in der ersten Kartusche verkantet, wodurch zumindest die notwendige Kraft zum Antreiben des Lagerungs- und Mischsystems reduziert wird, ein gleichmäßigerer Austrag des gemischten Knochenzementteigs erreicht wird und die Gefahr einer vollständigen Blockade des Lagerungs- und Mischsystems reduziert oder verhindert wird.

Dabei kann vorgesehen sein, dass die dritte Kartusche und/oder der dritte Austragskolben die gleichen Merkmale wie die zweite Kartusche und/oder der zweite Austragskolben nach einem der vorangehenden Lagerungs- und Mischsystemen aufweist.

Dies gilt insbesondere auch in Bezug auf die Wechselwirkung der dritten Kartusche und/oder des dritten Austragskolbens zu anderen Bauteilen erfindungsgemäßer Lagerungs- und Mischsysteme. Hieraus ergeben sich die gleichen Vorteile, wie für die zweite Kartusche und/oder den zweiten Austragskolben.

Ferner kann vorgesehen sein, dass zumindest eine vierte röhrenförmige Kartusche innerhalb der ersten röhrenförmigen Kartusche angeordnet ist, wobei die zumindest eine vierte Kartusche mit ihrer Außenwand an der Innenwand der ersten Kartusche anliegt und an der Innenwand der ersten Kartusche befestigt ist, wobei in der zumindest einen vierten Kartusche die zweite, die dritte, eine vierte und/oder jeweils zumindest eine weitere Ausgangskomponente des Knochenzements enthalten ist und in der zumindest einen vierten Kartusche jeweils ein vierter Austragskolben angeordnet ist, wobei mit dem vierten Austragskolben die zweite, die dritte, die vierte und/oder die jeweilige weitere Ausgangskomponente aus der zumindest einen vierten Kartusche durch eine gegenüberliegende Öffnung in der zumindest einen vierten Kartusche im Bereich des Kartuschenkopfs der ersten Kartusche auszutreiben ist, wobei die Pressvorrichtung vom Kartuschenkopf aus gesehen hinter dem oder den vierten Austragskolben angeordnet ist und die Pressvorrichtung zumindest eine Klemmkante zum Zusammenpressen der zumindest einen vierten Kartusche aufweist, wobei die Pressvorrichtung derart in Richtung des Kartuschenkopfs vortreibbar ist, dass während der Bewegung der Pressvorrichtung die zumindest eine vierte Kartusche axial fortlaufend zusammengepresst wird und dabei der erste Austragskolben, der zweite Austragskolben, der dritte Austragskolben und der vierte oder die vierten Austragskolben in Richtung des Kartuschenkopfs vorgetrieben werden.

Auch hierbei kann bevorzugt vorgesehen sein, dass die zumindest eine vierte Kartusche und/oder der oder die jeweiligen vierten Austragskolben die gleichen Merkmale wie die zweite Kartusche und/oder der zweite Austragskolben erfindungsgemäßer Lagerungs- und Mischsysteme aufweisen.

Dies gilt insbesondere auch in Bezug auf die Wechselwirkung der zumindest einen vierten Kartusche und/oder des vierten Austragskolbens oder der jeweiligen vierten Austragskolben zu anderen Bauteilen erfindungsgemäßer Lagerungs- und Mischsysteme. Auch kann erfindungsgemäß vorgesehen sein, dass die zumindest eine vierte Kartusche zusammen mit der zweiten und dritten Kartusche symmetrisch bezüglich der Achse der ersten Kartusche angeordnet sind, um eine gleichmäßige Durchmischung aller Ausgangskomponenten und eine gleichmäßige Krafteinwirkung auf die Kartuschen und die Pressvorrichtung zu erreichen.

Die der vorliegenden Erfindung zugrundeliegenden Aufgaben werden auch gelöst durch ein Verfahren zur Vermischung der Ausgangskomponenten eines pastenförmigen Zementteigs, insbesondere eines pastenförmigen Mehrkomponenten-Polymethylmethacrylat-Knochenzementteigs, mit einem erfindungsgemäßen Lagerungs- und Mischsystem mit den folgenden nacheinander ablaufenden Schritten:
a) Entfernen des Kartuschenkopfs von der ersten Kartusche oder Öffnen der Öffnungen der ersten Kartusche und der zweiten Kartusche,
b) Aufsetzen und Verbinden eines Austragsrohrs mit der Vorderseite der ersten Kartusche,
c) Einsetzen der ersten Kartusche in eine Auspressvorrichtung, die Auspressvorrichtung aufweisend einen axial vortreibbaren Stößel zum Vortreiben der Pressvorrichtung im Inneren der ersten Kartusche in Richtung des Austragsrohrs,
d) Auspressen der Ausgangskomponenten mit Hilfe der Auspressvorrichtung durch axiales Vortreiben des Stößels, wobei die Pressvorrichtung mit dem Stößel in Richtung des Austragsrohrs vorgetrieben wird, dabei der erste Austragskolben von der Pressvorrichtung in Richtung des Austragsrohrs geschoben wird, die Klemmkante der Pressvorrichtung die Wand der zweiten Kartusche zur Innenwand der ersten Kartusche presst, dabei die verformte Wand der zweiten Kartusche den zweiten Austragskolben in der zweiten Kartusche in Richtung des Austragsrohrs schiebt, wodurch die Ausgangskomponenten des Zementteigs beider Kartuschen in das Austragsrohr gedrückt werden, wobei die Ausgangskomponenten im Austragsrohr zu dem pastenförmigen Zementteig gemischt werden und der gemischte Zementteig aus einer Austragsöffnung des Austragsrohrs ausfließt.

Dabei kann vorgesehen sein, dass in Schritt a) die dritte Kartusche nach einer der oben genannten bevorzugten Ausführungen geöffnet wird, und bevorzugt auch die zumindest eine vierte Kartusche geöffnet wird, und in Schritt d) beim Vortreiben der Pressvorrichtung mit dem Stößel in Richtung des Austragsrohrs die Klemmkante oder eine weitere Klemmkante der Pressvorrichtung die Wand der dritten Kartusche zur Innenwand der ersten Kartusche presst, dabei die verformte Wand der dritten Kartusche den dritten Austragskolben in der dritten Kartusche in Richtung des Austragsrohrs schiebt und bevorzugt beim Vortreiben der Pressvorrichtung mit dem Stößel in Richtung des Austragsrohrs die Klemmkante oder eine oder mehrere weitere Klemmkante(n) der Pressvorrichtung auch die jeweilige Wand der zumindest einen vierten Kartusche zur Innenwand der ersten Kartusche presst beziehungsweise pressen, dabei die verformte Wand oder die verformten Wände der zumindest einen vierten Kartusche den jeweiligen vierten Austragskolben in der zumindest einen vierten Kartusche in Richtung des Austragsrohrs schiebt.

Hiermit können mehrere Ausgangskomponenten zu einem Zementteig gemischt werden oder es kann ein symmetrischerer Krafteintrag auf die Pressvorrichtung wirken, so dass es weniger leicht zu einer Verkantung oder Behinderung der Pressvorrichtung kommt.

Ferner kann vorgesehen sein, dass die Auspressvorrichtung manuell, mit Druckluft oder mit einem Motor angetrieben wird, wobei durch die manuelle Kraft, die Druckluft oder den Motor der Stößel in Richtung des Austragsrohrs vorgetrieben wird.

Manuell antreibbare Auspressvorrichtungen sind erfindungsgemäß bevorzugt, da sie weder an eine Druckluftquelle oder eine Energiequelle angeschlossen werden müssen, noch eine solche enthalten müssen.

Es kann vorgesehen sein, dass der Stößel der Auspressvorrichtung auf die von den Austragskolben abgewandte Seite der Pressvorrichtung drückt und die Austragskolben über die Pressvorrichtung angetrieben werden.

Der Erfindung liegt die überraschende Erkenntnis zugrunde, dass es auch durch die Anordnung zumindest einer inneren zweiten Kartusche in einer größeren äußeren ersten Kartusche, wobei die innere zweite Kartusche mit einer Pressvorrichtung, die innerhalb der äußeren ersten Kartusche läuft und dabei sowohl die Austragskolben in den Kartuschen vorschiebt als auch die Wand der inneren zweiten Kartusche zusammendrückt und damit aus dem Weg des Stößels der Auspressvorrichtung drückt, gelingt, eine Lagerungs- und Mischvorrichtung für einen Mehrkomponenten-Knochenzement bereitzustellen, mit dem auch eine geringfügige Menge zumindest einer zweiten Ausgangskomponente mit einer ersten Hauptausgangskomponente homogen und reproduzierbar mit dem gewünschten Mischungsverhältnis gemischt werden kann. Das Lagerungs- und Mischsystem ist dabei auch zur Lagerung der Ausgangskomponenten geeignet und kann zudem kostengünstig gefertigt werden. Trotz der kostengünstigen Ausführung ist die Lagerungs- und Mischvorrichtung einfach einzusetzen und kann auch mit manuell angetriebenen Auspressvorrichtungen verwendet werden. Zugleich ist die Gefahr für eine Blockade der Vorrichtung gering, so dass eine besonders betriebssichere Vorrichtung bereitgestellt wird. Die Lagerungs- und Mischvorrichtung kann aus kostengünstigen Materialien hergestellt werden. Die Abmessungen der erfindungsgemäßen Lagerungs- und Mischvorrichtung können dabei und dadurch derart kleinteilig gestaltet werden, dass auch bei der Verwendung der besonders zähen Ausgangskomponenten beziehungsweise der besonders zähen Haupt-Ausgangskomponente noch ein manuelles Auspressen und Mischen möglich ist. Beim Vortrieb der zähen Ausgangskomponenten durch einen statischen Mischer müssen nämlich erheblich Kräfte aufgebracht werden.

Ferner wurde überraschend gefunden, dass auf diese Weise eine schmale Kartusche mit nur einer Pressvorrichtung als Antrieb zum Vortreiben der Austragskolben zum Vortreiben der beiden Ausgangskomponenten verwendet werden kann, mit der auch gleichzeitig die Wand oder die Wände der zumindest einen inneren Kartusche aufgetrennt und seitlich weggedrückt werden kann. Dadurch kann die Kraft, die notwendig ist, um die Ausgangskomponenten zu mischen und auszutreiben, minimiert werden, so dass eine mit manueller Kraft anzutreibende Auspressvorrichtung zusammen mit dem Lagerungs- und Mischsystem eingesetzt werden kann, um die Ausgangskomponenten aus der Kartusche auszutreiben und miteinander zu mischen.

Die Erfindung basiert auf der Idee, in einer röhrenförmigen äußeren ersten Kartusche zumindest eine zweite kleinere innere zweite Kartusche vorzusehen, die einen kleineren Querschnitt als die größere äußere erste Kartusche besitzt, wobei in beiden Kartuschen jeweils ein axial beweglicher Austragskolben angeordnet ist, und dass der größere Austragskolben mit einer Pressvorrichtung verbunden ist, die mit einer Klemmkante die Wand der zumindest einen kleineren inneren zweiten Kartusche bei der Bewegung des Klemmkörpers in Richtung Kartuschenkopf so gegen die Wand der größeren ersten Kartusche drückt, dass die Kartuschenwand deformiert wird und bei der Vorwärtsbewegung der Pressvorrichtung der Austragskolben der kleineren zweiten Kartusche durch die deformierte Wand beziehungsweise durch die fortlaufende Deformation der Wand in Richtung Kartuschenkopf bewegt wird. Zeitgleich wird bei der Vorwärtsbewegung auch der Austragskolben der größeren ersten Kartusche in Richtung Kartuschenkopf bewegt. Die deformierte, gequetschte Kartuschenwand der inneren zweiten Kartusche rutscht durch eine äußere seitliche Öffnung der Pressvorrichtung bei der Vorwärtsbewegung der Pressvorrichtung. Es verbleiben bei dem Austrag der pastenförmigen zweiten Ausgangskomponente aus der kleineren zweiten Kartusche durch Vorwärtsbewegung des zweiten Austragskolbens praktisch keine Reste der pastenförmigen zweiten Ausgangskomponente in der deformierten kleinen zweiten Kartusche. Wichtig ist dabei, dass die Pressvorrichtung auf ihrer Rückseite eine zentrale Auflagefläche für einen Stößel besitzt, wobei die Auflagefläche außerhalb der Öffnung der Pressvorrichtung angeordnet ist, um eine ungestörte Auspressbewegung des Stößels zu ermöglichen. Weiterhin basiert die Erfindung auf der überraschenden Beobachtung, dass hochviskose Zementpasten als Ausgangskomponenten aus einer Kartusche mit einem Austragsrohr und darin angeordneten statischen Mischern durch manuell zu betätigende Auspressvorrichtungen ausgepresst werden können, wenn der Querschnitt der röhrenförmigen Kartusche gleich oder kleiner 25 mm ist.

Erfindungsgemäß ist daher, dass der Innendurchmesser der ersten Kartusche kleiner, gleich 25 mm ist und bevorzugt kleiner, gleich 20 mm ist.

Die Erfindung basiert auf der Idee, zur Minimierung des Strömungswiderstands beim Austrag nur eine zylindrische äußere Kartusche, anstelle von mehreren side-by-side-Kartuschen oder Koaxial-Kartuschen, für die separate Lagerung der beiden pastenförmigen Ausgangskomponenten einzusetzen. Durch das Wegdrücken der Wand der inneren Kartuschen mit Hilfe der Pressvorrichtung können auch kleinere Mengen des PMMA-Knochenzements verwendet werden und auch schmalere Kartuschen mit Innenräumen mit kleineren Innendurchmessern sind noch auspressbar.

Eigene Versuche zeigten, dass während des Auspressvorgangs der Kartuschen auf Grund der hohen Viskosität der pastenförmigen Ausgangskomponenten ein sehr großer Druckabfall am statischen Mischer im Austragsrohr stattfindet. Die Versuche zeigten weiterhin, dass bei einem konischen Austragsrohr und einer Gesamtlänge von ca. 17 cm und mit einem Innendurchmesser von 11 mm am Kartuschenkopf und unter Verwendung von 10 statischen Mischelementen eine Auspresskraft größer 7 kN notwendig ist, um die hoch viskosen Zementpasten in einer für den medizinischen Anwender akzeptablen Austragsgeschwindigkeit auszupressen. Diese Untersuchungen haben zu den erfindungsgemäßen Vorrichtungen und den erfindungsgemäßen Verfahren geführt.

Weiterhin basiert die Erfindung auf der Beobachtung, dass hochviskoser Zementteig aus zylinderförmigen Kartuschen durch ein Austragsrohr mit statischem Mischer mit handelsüblichen manuell anzutreibenden Auspressvorrichtungen beziehungsweise Applikatoren in akzeptabler Zeit und mit akzeptablem, da manuell aufbringbarem Kraftaufwand ausgetragen werden kann, wenn der Austragskolben an seiner Stirnseite einen Durchmesser von maximal 25 mm hat. Mit dem erfindungsgemäßen Aufbau wird ein Lagerungs- und Mischsystem bereitgestellt, das derartig kleine Durchmesser für die Anwendung hochviskoser Ausgangskomponenten realisieren kann. Dabei kann die erste Kartusche beziehungsweise können die Hohlräume dennoch ohne zu großen Aufwand mit den Ausgangskomponenten befüllt werden.

Ein beispielhaftes und besonders bevorzugtes erfindungsgemäßes Lagerungs- und Mischsystem für pastenförmigen Zweikomponenten-Polymethylmethacrylat-Knochenzement ist zusammengesetzt aus
a) einer röhrenförmigen ersten Kartusche, in deren Innenraum eine erste Zementpaste als erste Ausgangkomponente (beziehungsweise Haupt-Ausgangskomponente) gelagert ist,
b) mindestens einer im Innenraum der ersten Kartusche angebrachten röhrenförmigen zweiten Kartusche, die mit der Innenwand der ersten Kartusche über die gesamte Länge der zweiten Kartusche verbunden ist, wobei im Innenraum der zweiten Kartusche eine zweite Zementpaste als zweite Ausgangskomponente gelagert ist,
c) einem von der ersten Kartusche und der zweiten Kartusche lösbaren Kartuschenkopf, der ein Ende (oder jeweils eine Öffnung an dem Ende) der ersten Kartusche und ein Ende der zweiten Kartusche verschließt,
d) einem Austragsrohr mit einem Befestigungsmittel, zur Befestigung an der ersten Kartusche, wobei in dem Austragsrohr ein statischer Mischer angeordnet ist,
e) einem ersten Austragskolben, der in dem Innenraum der ersten Kartusche axial beweglich angeordnet ist,
f) einem zweiten Austragskolben, der in dem Innenraum der zweiten Kartusche axial beweglich angeordnet ist,
g) einer mit dem ersten Austragskolben verbundenen Pressvorrichtung, die eine schräge Klemmkante besitzt, die im Winkel von mindestens 40° senkrecht zur Längsachse in Richtung der Innenwand der ersten Kartusche geneigt ist, wobei die Klemmkante die Wand der zweiten Kartusche gegen die Innenwand der ersten Kartusche quetscht, wobei die Klemmkante mindestens 30 Prozent der Fläche des Querschnitts der zweiten Kartusche überdeckt,
h) wobei ein Spalt zwischen der Außenseite der Pressvorrichtung und der Innenwand der ersten Kartusche ausgebildet ist, wobei die Spaltfläche beziehungsweise das Spaltmaß gleich oder größer der Querschnittsfläche der gequetschten zweiten Kartusche ist,
i) die Rückseite der Pressvorrichtung als Auflagefläche für einen Stößel einer Auspressvorrichtung ausgebildet ist,
j) der zweite Austragskolben derart in der zweiten Kartusche angeordnet ist, dass das die Unterseite des zweiten Austragskolbens vor der Pressvorrichtung in Richtung des Kartuschenkopfs angeordnet ist, und
k) bei Bewegung der Pressvorrichtung in Richtung Kartuschenkopf der Pressvorrichtung durch Quetschung der zweiten Kartusche, die deformierte Wand der zweiten Kartusche auf die Unterseite des zweiten Austragskolbens presst und den zweiten Austragskolben in Richtung Kartuschenkopf bewegt.

Die Pressvorrichtung ist als eine Art Klemmkörper ausgebildet, mit dem über die Klemmkante die zweite Kartusche verformt beziehungsweise gegen die Innenwand der ersten Kartusche gequetscht wird.

Erfindungsgemäß kann vorgesehen sein, dass zwei zweite Kartuschen, beziehungsweise eine zweite und eine dritte Kartusche im Innenraum der ersten Kartusche angeordnet sind. Dadurch ist es möglich eine dritte Zementkomponente als Ausgangskomponente, zum Beispiel pharmazeutische Wirkstoffe, mit in den Zementteig einzubringen. Das kann vorteilhaft sein, wenn pharmazeutische Wirkstoffe zum Beispiel gegenüber speziellen Komponenten der Zementpasten, wie zum Beispiel Peroxide, über den Zeitraum von Befüllung bis zur Anwendung des Pastenzements beziehungsweise des gemischten Zementteigs instabil sind. Weiterhin können damit auch Zementpasten mit komplexen, aus mehreren Komponenten bestehenden Initiatorsystemen in dem Lagerungs- und Mischsystem gelagert werden, bei denen die Initiatorkomponenten getrennt gelagert werden müssen. Aus Gründen eines symmetrischen Krafteintrags in die Pressvorrichtung beim Vortreiben der Pressvorrichtung in dem Lagerungs- und Mischsystem kann es auch bei der Verwendung eines Zweikomponenten-Knochenzements sinnvoll sein, wenn zwei innere zweite beziehungsweise eine innere zweite und eine innere dritte Kartusche in der ersten Kartusche angeordnet sind. Dadurch kann beispielsweise einem Verkanten der Pressvorrichtung vorgebeugt werden.

Die Pressvorrichtung oder zumindest die Klemmkante ist bevorzugt aus Metall und/oder Kunststoff und/oder glasfaserverstärktem Kunststoff gefertigt. Bevorzugt besteht die Pressvorrichtung oder zumindest die Klemmkante aus Stahl, einer Aluminiumlegierung, einer Zinklegierung, Polyamid, glasfaserverstärktem Polyamid, Polyetherketon oder Polysulfon oder Kombinationen dieser Werkstoffe. Es kommen auch Keramiken und Karbonfasern enthaltende Komposite zum Aufbau der Pressvorrichtung oder zumindest der Klemmkante in Betracht.

Die pastenförmigen Ausgangskomponenten enthalten das sehr flüchtige, radikalisch polymerisierbare Monomer Methylmethacrylat. Für eine Lagerung der Ausgangskomponenten ist es daher unerlässlich, dass die Kartuschen, der Kartuschenkopf und die Austragskolben aus Kunststoffen gefertigt sind, die eine gute Diffusionsbarriere für Methylmethacrylat darstellen. Erfindungsgemäß kann daher vorgesehen sein, dass die Kartuschen, der Kartuschenkopf, die Trennwand und die Austragskolben aus Kunststoff gefertigt sind, wobei als Kunststoffe Polyethylen-covinylalkohol (EVOH), Polybutylenterephthalat (PBT), Polyethylenterephthalat (PET) und Poly-methacrylsäuremethylester-co-acrylnitril bevorzugt werden. Es zusätzlich auch möglich, auf die nicht die Ausgangskomponenten berührenden Teile diffusionsdichte Metallschichten, Metall- oder Halbmetalloxidschichten oder Kunststoffschichten aufzubringen. Als Metallschichten kommen insbesondere Aluminiumschichten in Betracht. Als Halbmetalloxidschichten sind insbesondere Siliziumdioxid-Schichten geeignet.

Die Kartusche hat beispielsweise an einem Ende ein Befestigungselement für eine Auspressvorrichtung und am gegenüberliegenden Ende an der mindestens ein Außengewinde und/oder ein Innengewinde und/oder mindestens eines Elements eines Bajonettverschlusses und/oder ein mindestens ein Rastelement eines Rastverschlusses als Verbindungselement.

Der Kartuschenkopf kann aus einer gummielastischen Platte und einer aus Kunststoff gefertigten Überwurfkappe gebildet sein, wobei die Überwurfkappe die gummielastische Platte nach oben durch einen überragenden Rand blockiert, und wobei in der gummielastische Platte zwei Öffnung angeordnet sind, die durch einen Stopfen verschlossen ist.

In einer ersten Ausgestaltungsform sind eine Kunststoffplatte mit zwei mit Stopfen verschließbare Öffnungen auf oder unter der gummielastischen Platte im Kartuschenkopf angeordnet.

Die Überwurfkappe besitzt als Verbindungselement ein Innengewinde oder ein Außengewinde oder ein Bajonettverschluss oder Rastelemente für einen rastenden Verschluss.

Erfindungsgemäß ist in einem Austragsrohr ein statischer Mischer angeordnet. An der Basis des Austragsrohrs ist beziehungsweise sind als Verbindungsmittel ein Innengewinde und/oder ein Außengewinde und/oder Elemente eines Bajonettverschlusses und/oder Rastelemente eines Rastverschlusses angebracht.

In einer alternativen Ausgestaltungsform ist die Stirnfläche der Kartusche als Kartuschenkopf ausgebildet, wobei zwei durch Stopfen verschließbare Öffnungen die Stirnfläche der Kartusche durchbrechen.

Erfindungsgemäß ist beispielsweise auch ein Verfahren zur Vermischung der pastenförmigen Ausgangskomponenten des pastenförmigen Polymethylmethacrylat-Knochenzements mit dem Lagerungs- und Mischsystem. Das Verfahren ist durch folgende nacheinander ablaufende Schritte gekennzeichnet:
a) Entfernen des Kartuschenkopfs oder der Verschlüsse von der ersten Kartusche, beziehungsweise von der ersten und zweiten Kartusche,
b) Verbinden des Austragsrohrs, das einen statischen Mischer enthält, mit der geöffneten ersten Kartusche,
c) Verbinden der ersten Kartusche mit einer manuell zu betätigenden Auspressvorrichtung,
d) manuelle Betätigung der Auspressvorrichtung, wobei der Stößel der Auspressvorrichtung auf die Auflagefläche der Pressvorrichtung in Richtung des Austragsrohrs drückt, wobei die Pressvorrichtung die zweite Kartusche gegen die Wand der ersten Kartusche quetscht und den zweiten Austragskolben in Richtung Austragsrohr bewegt und wobei gleichzeitig die Pressvorrichtung den ersten Austragskolben in Richtung des Austragsrohrs bewegt,
e) Auspressen der ersten Zementpaste (der ersten Ausgangskomponente) aus der ersten Kartusche durch axiale Bewegung des ersten Austragskolbens in Richtung des Austragsrohrs,
f) Auspressen der zweiten Zementpaste (der zweiten Ausgangskomponente) aus der ersten Kartusche durch axiale Bewegung des ersten Austragskolbens in Richtung des Austragsrohrs aus der zweiten Kartusche,
g) Vermischen der ersten Zementpaste und der zweiten Zementpaste mit dem statischen Mischer im Austragsrohr und
h) Austrag der gemischten Zementpaste (dem Knochenzementteig) aus dem Austragsrohr.

In einer Variante dieses Verfahrens wird in den Schritten c) und d) anstelle der manuell angetriebenen Auspressvorrichtung eine durch Druckluft oder durch elektrischen Strom angetriebene Auspressvorrichtung verwendet.

Im Folgenden werden weitere Ausführungsbeispiele der Erfindung anhand von siebzehn schematisch dargestellten Figuren erläutert, ohne jedoch dabei die Erfindung zu beschränken. Dabei zeigt:
Figur 1: eine schematische Querschnittansicht eines ersten beispielhaften erfindungsgemäßen Lagerungs- und Mischsystems;
Figur 2: eine schematische perspektivische Ansicht des geöffneten erfindungsgemäßen Lagerungs- und Mischsystems nach Figur 1;
Figur 3: eine schematische Querschnittansicht durch das erfindungsgemäße Lagerungs- und Mischsystem nach den Figuren 1 und 2 unmittelbar vor der Applikation des PMMA-Knochenzements, an dem ein Austragsrohr befestigt ist;
Figur 4: eine schematische Querschnittansicht durch das erfindungsgemäße Lagerungs- und Mischsystem nach Figur 3, während des Mischvorgangs;
Figur 5: eine vergrößerte schematische Querschnittansicht des Antriebs des erfindungsgemäßen Lagerungs- und Mischsystems nach Figur 4;
Figur 6: eine schematische Querschnittansicht eines zweiten, alternativen erfindungsgemäßen Lagerungs- und Mischsystems;
Figur 7: eine schematische perspektivische Ansicht des geöffneten zweiten erfindungsgemäßen Lagerungs- und Mischsystems nach Figur 6;
Figur 8: eine schematische Querschnittansicht durch das zweite erfindungsgemäße Lagerungs- und Mischsystem nach den Figuren 6 und 7 unmittelbar vor der Applikation des PMMA-Knochenzements, bei dem ein Austragsrohr befestigt ist;
Figur 9: eine schematische Querschnittansicht durch das zweite erfindungsgemäße Lagerungs- und Mischsystem nach Figur 8, während des Mischvorgangs;
Figur 10: eine vergrößerte schematische Querschnittansicht des Antriebs des zweiten erfindungsgemäßen Lagerungs- und Mischsystems nach Figur 9;
Figur 11: zwei schematische perspektivische Ansichten einer Pressvorrichtung für ein erfindungsgemäßes Lagerungs- und Mischsystem mit zwei inneren Kartuschen;
Figur 12: eine schematische perspektivische Ansicht einer Pressvorrichtung für ein erfindungsgemäßes Lagerungs- und Mischsystem mit einer inneren Kartusche;
Figur 13: eine schematische perspektivische Ansicht auf ein drittes, alternatives erfindungsgemäßes Lagerungs- und Mischsystem;
Figur 14: eine schematische perspektivische Querschnittansicht des dritten erfindungsgemäßen Lagerungs- und Mischsystems nach Figur 13 mit eingeschobener Pressvorrichtung;
Figur 15: zwei schematische perspektivische Schnittansichten des vorderen Bereichs des dritten erfindungsgemäßen Lagerungs- und Mischsystems nach den Figuren 13 und 14;
Figur 16: eine Vergrößerung eines Teils des dritten erfindungsgemäßen Lagerungs- und Mischsystems nach den Figuren 13 bis 15 als schematische perspektivische Querschnittansicht; und
Figur 17: eine Explosionsdarstellung (oben) und eine Schnittansicht (unten) des vorderen Bereichs des dritten erfindungsgemäßen Lagerungs- und Mischsystems nach Figur 15.

In den Figuren werden der Einfachheit halber an gelegentlich für gleiche und gleichartige Bauteile teilweise die gleichen Bezugszeichen verwendet.

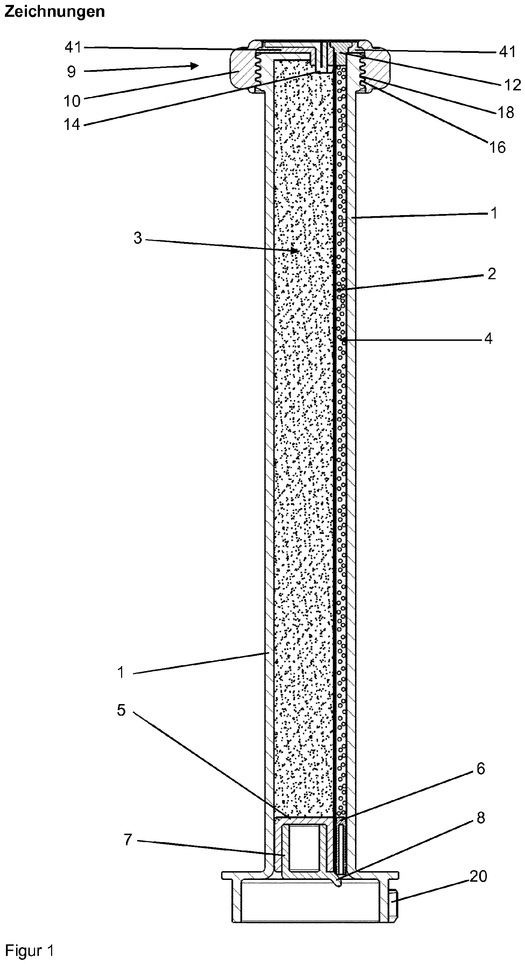

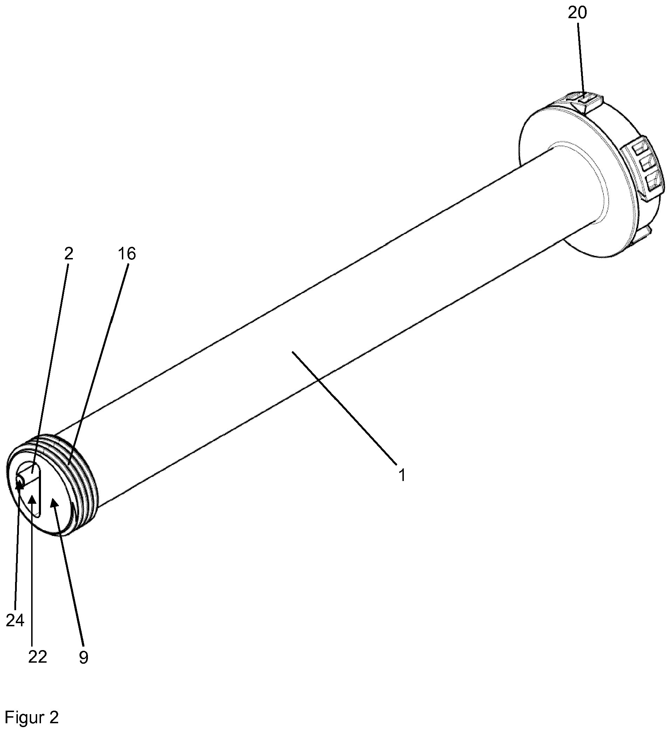

Die Figuren 1 bis 5 zeigen eine erste beispielhafte Ausführung eines erfindungsgemäßen Lagerungs- und Mischsystems. Figur 1 zeigt dabei eine schematische Querschnittansicht des erfindungsgemäßen Lagerungs- und Mischsystems und Figur 2 eine schematische perspektivische Ansicht des geöffneten erfindungsgemäßen Lagerungs- und Mischsystems nach Figur 1. Das Lagerungs- und Mischsystem weist eine äußere erste Kartusche 1 auf, in der eine innere zweite Kartusche 2 an der Innenwand der ersten Kartusche 1 über die gesamte Länge der ersten Kartusche 1 befestigt ist. Beide Kartuschen 1, 2 sind aus dem gleichen Material gefertigt. Die Wandstärke der inneren zweiten Kartusche 2 entspricht etwa einem Viertel der Wandstärke der äußeren ersten Kartusche 1. Der Innenraum der ersten Kartusche 1 ist bis auf den Raum, den die zweite Kartusche 2 einnimmt, mit einer ersten pastösen Ausgangskomponente 3 eines PMMA-Knochenzements gefüllt. Der Innenraum der zweiten Kartusche 2 ist mit einer zweiten pastösen Ausgangskomponente 4 des Zweikomponenten-PMMA-Knochenzements gefüllt. Die Innenräume der Kartuschen 1, 2 sind auf deren Rückseite (in den Figuren 1 und 3 bis 5 unten und in Figur 2 rechts oben) durch einen ersten Austragskolben 5 in der ersten Kartusche 1 und einen zweiten Austragskolben 6 in der zweiten Kartusche 2 begrenzt, wobei die Austragskolben 5, 6 die Innenräume der Kartuschen 1, 2 nach außen fluiddicht abschließen. Der Austragskolben 5 der ersten Kartusche 1 weist dementsprechend eine seitliche Ausnehmung auf, so dass er über die zweite Kartusche 2 gleiten kann, aber auch dort dicht abschließt. Der Austragskolben 6 der zweiten Kartusche 2 weist passend zum kleineren Innenraum einen geringeren Durchmesser auf als der Austragskolben 5 der ersten Kartusche 1.

Der Innenraum der zweiten Kartusche 2 ist zylindrisch mit kreisförmiger Grundfläche geformt. Der Innenraum der ersten Kartusche 1 ist ebenfalls zylindrisch mit kreisförmiger Grundfläche geformt, wobei die zweite Kartusche 2 einen Teil des Innenraums der ersten Kartusche 1 einnimmt und so eine Brechung der kreisförmig zylindrischen Symmetrie des Innenraums der ersten Kartusche 1 bewirkt. Der Austragskolben 5 der ersten Kartusche 1 weist an seiner Rückseite beziehungsweise bodenseitig (in den Figuren 1 und 3 bis 5 unten) eine Vertiefung auf, in die eine Pressvorrichtung 7 eingesteckt ist. Die Pressvorrichtung 7 weist an ihrer Rückseite eine Klemmkante 8 auf, die in den durch die zweite Kartusche 2 belegten Raum im Innenraum der ersten Kartusche 1 greift, wenn die Pressvorrichtung 7 innerhalb der ersten Kartusche 1 nach vorne (in den Figuren 1 und 3 bis 5 nach oben) getrieben wird. Die Klemmkante 8 weist eine abgeschrägte Oberfläche auf, die in Richtung senkrecht zur Zylinderachse der zweiten Kartusche 2 geneigt ist. Die zweite Kartusche 2 hat ein Volumen von etwa einem Zwanzigstel der ersten Kartusche 1. Dementsprechend wird der Zementteig mit einem Mischungsverhältnis von etwa 20 zu 1 aus den Ausgangskomponenten 3, 4 gemischt. Aufgrund der zylindrischen Symmetrie der Innenräume der Kartuschen 1, 2 bleibt das Mischungsverhältnis während des Auspressvorgangs konstant.

Die Klemmkante 8 oder die gesamte Pressvorrichtung 7 bestehen aus einem Material und/oder sind derart geformt, dass die Pressvorrichtung 7 oder zumindest die Klemmkante 8 härter oder fester ist als die Wand der zweiten Kartusche 2. Bevorzugt besteht die Klemmkante 8 und die gesamte Pressvorrichtung 7 aus einem Metall, insbesondere einer Aluminium-Legierung, oder einem festen Kunststoff, der zumindest härter, fester und/oder zäher ist als das Material der Wand der zweiten inneren Kartusche 2.

Die Austragskolben 5, 6 sind axial in Längsrichtung in den Innenräumen der Kartuschen 1, 2 in Richtung eines Kartuschenkopfs 9 der Kartuschen 1, 2 beweglich gelagert (in den Figuren 1 und 3 bis 5 von unten nach oben). In dem Kartuschenkopf 9 sind eine Öffnung 22 der ersten Kartusche 1 und eine Öffnung 24 der zweiten Kartusche 2 vorgesehen (siehe Figur 2). Im Bereich des Kartuschenkopfs 9 ist im Lagerungszustand des Lagerungs- und Mischsystems (siehe Figur 1) eine Überwurfmutter 10 aufgeschraubt, mit der eine gummielastische Platte 41 gehalten wird, die die beiden Öffnungen 22, 24 begrenzt und die einen Teil des Kartuschenkopfs 9 bildet. Zwei Stopfen 12, 14 sind in die Öffnungen 22, 24 eingesteckt, die die Öffnungen 22, 24 und damit die Innenräume der Kartuschen 1, 2 an der Vorderseite (in den Figur 1 oben in Figur 2 unten links, in Richtung des Betrachters) fluiddicht verschließen. Die Öffnung 22 der ersten Kartusche 1 ist derart platziert, dass sie zu der Öffnung 24 der zweiten Kartusche 1 hin ausgerichtet beziehungsweise benachbart ist. In den Bereichen die weiter von der Öffnung 24 weg liegen, ist die erste Kartusche 1 im Bereich des Kartuschenkopfs 9 geschlossen. Hiermit wird erreicht, dass die zweite Ausgangskomponente 4 bereits beim Austritt aus der zweiten Kartusche 2 tiefer in den Strom der ersten Ausgangskomponente 3 gemischt wird. Hiermit wird eine bessere Durchmischung der beiden Ausgangskomponenten 3, 4 erzeugt.

An der Vorderseite der ersten Kartusche 1 ist außen ein Außengewinde 16 als Befestigungselement 16 vorgesehen, auf das die Überwurfmutter 10 aufgeschraubt werden kann beziehungsweise ist. Die Überwurfmutter 10 weist dazu ein passendes Innengewinde 18 als Gegenbefestigungselement 18 auf. An der Rückseite des Lagerungs- und Mischsystems ist ein Stutzen mit einem Anschluss 20 zur Befestigung einer Auspressvorrichtung (nicht gezeigt) vorgesehen. Die Auspressvorrichtung hält die äußere erste Kartusche 1 und weist einen Stößel auf, mit dem die Pressvorrichtung 7 in Richtung des Kartuschenkopfs 9 gedrückt werden kann. Die Auspressvorrichtung wird bevorzugt manuell angetrieben.

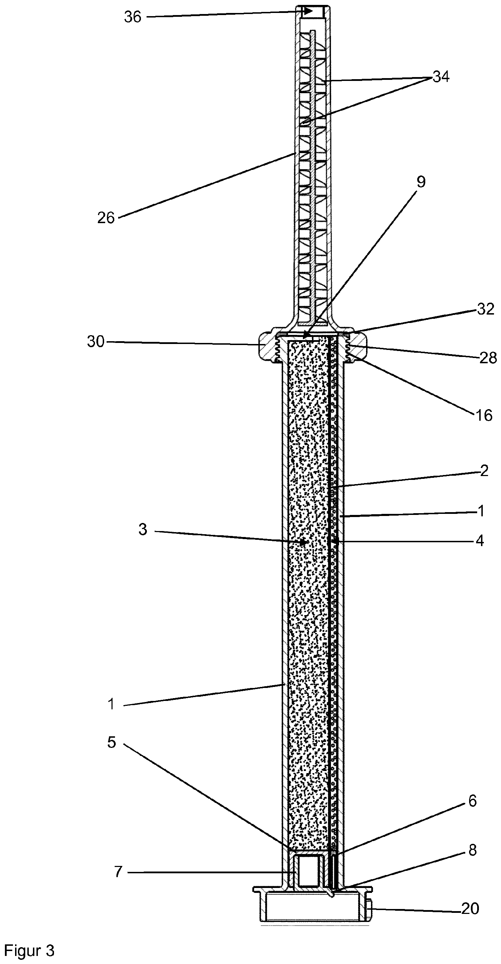

Figur 3 zeigt eine schematische Querschnittansicht durch das erfindungsgemäße Lagerungs- und Mischsystem nach den Figuren 1 und 2 unmittelbar vor der Applikation des PMMA-Knochenzements, bei dem ein Austragsrohr 26 an der Vorderseite der ersten Kartusche 1 befestigt ist. Um das Austragsrohr 26 an der ersten Kartusche 1 zu befestigen, wird die Überwurfmutter 10 abgeschraubt und werden die Stopfen 12, 14 entfernt und so die Kartuschen 1, 2 geöffnet. Anschließend wird auf das Außengewinde 16 das Austragsrohr 26 aufgeschraubt. Dazu ist ein zum Außengewinde 16 passendes Innengewinde 28 an dem Austragsrohr 26 im Bereich eines Stutzens 30 vorgesehen. Das Austragsrohr 26 ist mit einer umlaufenden Dichtung 32 gegen das Lagerungs- und Mischsystem beziehungsweise den Anschluss der ersten Kartusche 1 an dem Kartuschenkopf 9 abgedichtet, damit keine Ausgangskomponenten 3, 4 und kein gemischter Zementteig zwischen dem Austragsrohr 26 und dem Kartuschenkopf 9 nach außen gedrückt wird.

In dem Austragsrohr 26 sind zehn statische Mischer 34 angeordnet, die die Ausgangskomponenten 3, 4 miteinander durchmischen, wenn diese durch das Austragsrohr 26 gepresst werden. Dadurch entsteht ein gut durchmischter Zementteig, der über eine Austragsöffnung 36 an der Spitze des Austragsrohrs 26 ausgetragen beziehungsweise appliziert werden kann. Das Austragsrohr 26 kann Teil des erfindungsgemäßen Lagerungs- und Mischsystems sein.

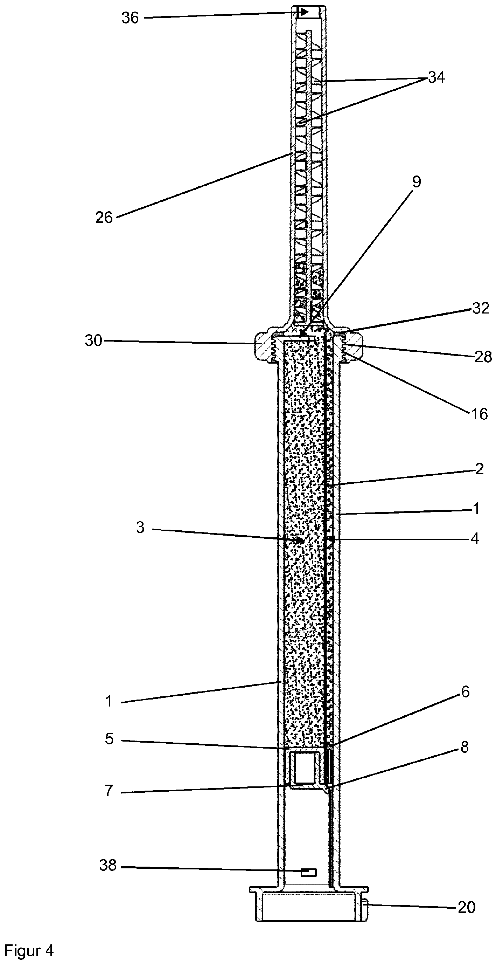

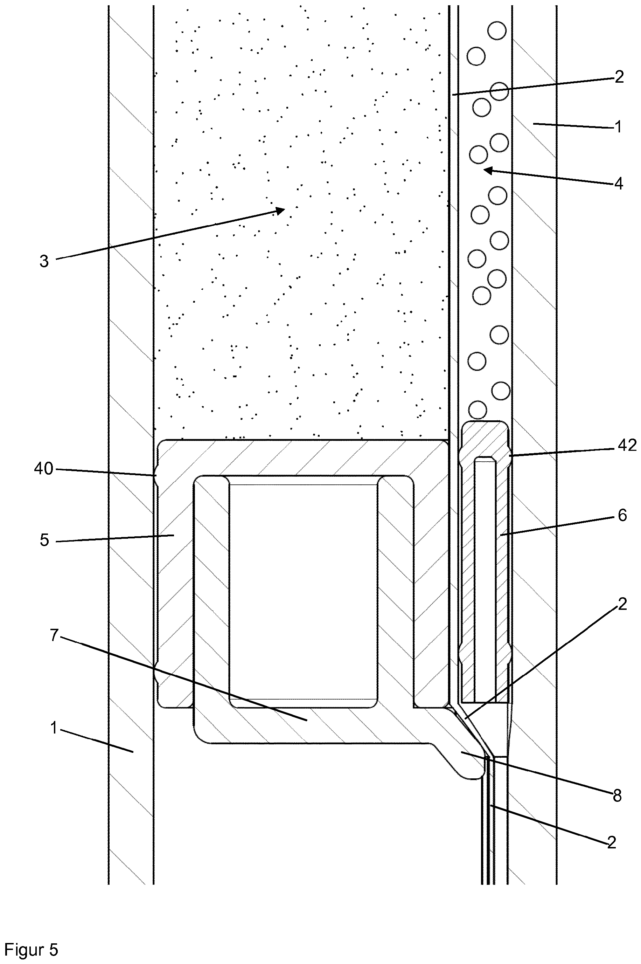

Wenn das Austragsrohr 26 an der ersten Kartusche 1 befestigt ist, wird das Lagerungs- und Mischsystem in die Auspressvorrichtung (nicht gezeigt) eingesetzt und über den Anschluss 20 mit der Auspressvorrichtung verbunden. Der Stößel der Auspressvorrichtung wird bodenseitig in das Lagerungs- und Mischsystem eingetrieben und drückt so bodenseitig auf die Pressvorrichtung 7. Da der erste Austragskolben 5 mit der Pressvorrichtung 7 verbunden ist, wird dieser von der Pressvorrichtung 7 in die erste Kartusche 1 eingeschoben und dabei die erste Komponente 3 aus der ersten Kartusche 1 in das Austragsrohr 26 gedrückt. Gleichzeitig wird die Wand der zweiten Kartusche 2 von der Klemmkante 8 in Richtung der Innenwand der ersten Kartusche 1 gedrückt. Durch die Verformung der Wand der zweiten Kartusche 2 wird der zweite Austragskolben 6 in Richtung des Kartuschenkopfs 9 gedrückt und so die zweite Ausgangskomponente 4 im Inneren der zweiten Kartusche 2 in das Austragsrohr 26 gepresst. Diese Situation ist in den Figuren 4 und 5 dargestellt. Figur 4 zeigt hierzu eine schematische Querschnittansicht durch das erfindungsgemäße Lagerungs- und Mischsystem nach Figur 3 während des Mischvorgangs und Figur 5 eine vergrößerte schematische Querschnittansicht des Antriebs des erfindungsgemäßen Lagerungs- und Mischsystems nach Figur 4.

In der Innenwand der äußeren ersten Kartusche 1 sind zwei gegenüberliegende Vertiefungen 38 als Rastmittel 38 vorgesehen. In dem ersten Austragskolben 5 sind an der äußeren Mantelfläche zwei passende Gegenrastmittel (nicht zu sehen) vorgesehen, die in die Vertiefungen 38 greifen können und den Austragskolben 5 dadurch in der Ausgangsposition halten, die zum Lagern der Ausgangskomponenten 3, 4 geeignet ist (siehe Figur 1). Die Rastung ist durch einen Druck auf die Rückseite des Austragskolbens 5 beziehungsweise auf die Rückseite der Pressvorrichtung 7 lösbar, so dass der erste Austragskolben 5 und damit die Pressvorrichtung 7 in Richtung des Kartuschenkopfs 9 bewegbar ist, wenn der Rastwiderstand überwunden wird.

An dem ersten Austragskolben 5 sind zwei umlaufende Erhebungen 40 als Dichtungen 40 vorgesehen, mit denen der Austragskolben 5 mit den Innenwänden der ersten Kartusche 1 abschließt. Ebenso sind an dem zweiten Austragskolben 6 zwei umlaufende Erhebungen 42 als Dichtungen 42 vorgesehen, mit denen der Austragskolben 6 mit den Innenwänden der zweiten Kartusche 2 abschließt. Mit diesen Dichtungen 40, 42 kann sichergestellt werden, dass der gesamte Inhalt der beiden Kartuschen 1, 2, also die beiden Ausgangskomponenten 3, 4 restlos ausgetrieben werden und so zum Herstellen einer PMMA-Knochenzement-Mischung mit dem gewünschten Verhältnis genutzt werden können. Die Erhebungen 40, 42 können auch durch Dichtungsringe aus Gummi gebildet werden. Dadurch, dass die Wand der zweiten Kartusche 2 erst nach dem Austreiben der zweiten Ausgangskomponente 4 mit dem zweiten Austragskolben 6 zusammengedrückt wird, können in den beim Zusammendrücken der Wand der zweiten Kartusche 2 entstehenden Falten keine Reste der zweiten Ausgangskomponente 4 zurückbleiben und so das Mischungsverhältnis im Zementteig nicht verfälscht werden.

Mit dem erfindungsgemäßen Aufbau des Lagerungs- und Mischsystems können auch geringe Mengen der zweiten Ausgangskomponente 4 im richtigen beziehungsweise gewünschten Mischungsverhältnis beigemischt werden. Herkömmliche Auspressvorrichtungen mit einem zentralen Stößel können dazu verwendet werden, den Zementteig zu mischen und auszutragen, da die Wand der zweiten Kartusche 2 nach außen in Richtung der ersten Kartusche 1 gedrückt wird und so der Bewegung des Stößels nicht im Wege ist.

Die äußere erste Kartusche 1 kann dabei derart schmal gestaltet werden, erfindungsgemäß bevorzugt mit einem Innendurchmesser von maximal 25 mm oder besonders bevorzugt mit einem Innendurchmesser von maximal 20 mm, dass die zähen Ausgangskomponenten 3, 4, insbesondere die zähe erste Ausgangskomponente 3, in das Austragsrohr 26 und durch die statischen Mischer 34 gedrückt werden kann, ohne dass der Widerstand der zähen Pasten 3, 4 derart groß würde, dass diese nicht mehr mit herkömmlichen, manuell angetriebenen Auspressvorrichtungen ausgetrieben werden könnten.

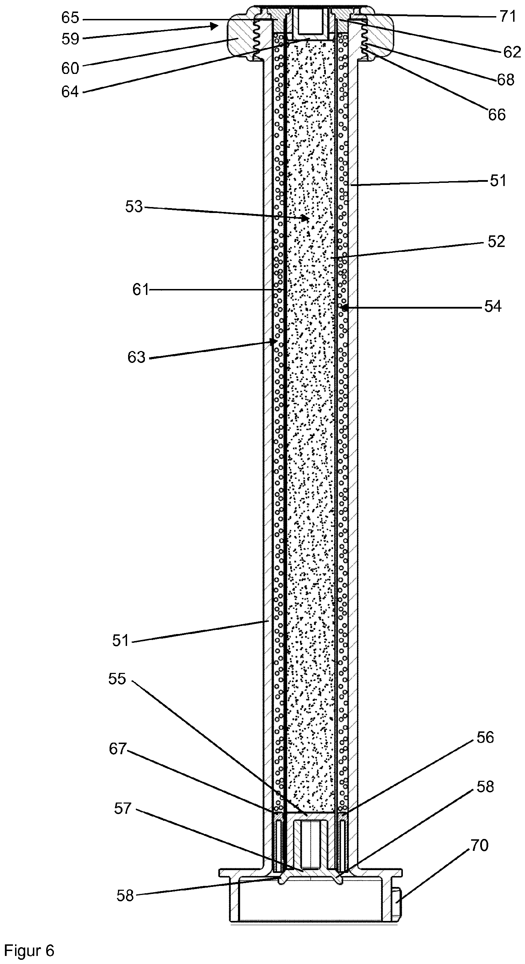

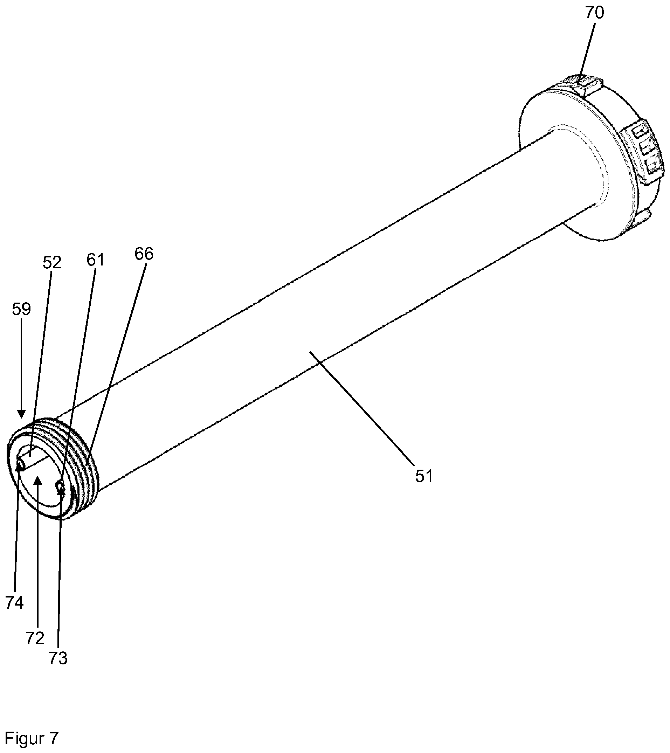

Die Figuren 6 bis 10 zeigen eine alternative zweite beispielhafte Ausführung eines erfindungsgemäßen Lagerungs- und Mischsystems. Figur 6 zeigt dabei eine schematische Querschnittansicht des erfindungsgemäßen Lagerungs- und Mischsystems und Figur 7 eine schematische perspektivische Ansicht des geöffneten erfindungsgemäßen Lagerungs- und Mischsystems nach Figur 6. Das Lagerungs- und Mischsystem weist eine äußere erste Kartusche 51 auf, in der eine innere zweite Kartusche 52 an einer Innenwand der ersten Kartusche 51 über die gesamte Länge der ersten Kartusche 51 befestigt ist. Zudem ist an der gegenüberliegenden Innenwand der ersten Kartusche 51 eine innere dritte Kartusche 61 über die gesamte Länge der ersten Kartusche 51 befestigt. Alle drei Kartuschen 51, 52, 61 sind aus dem gleichen Material gefertigt. Die Wandstärken der inneren zweiten Kartusche 52 und der inneren dritten Kartusche 61 entsprechen etwa einem Viertel der Wandstärke der äußeren ersten Kartusche 51. Der Innenraum der ersten Kartusche 51 ist bis auf den Raum, den die zweite Kartusche 52 und die dritte Kartusche 61 einnehmen, mit einer ersten pastösen Ausgangskomponente 53 eines Mehrkomponenten-PMMA-Knochenzements gefüllt. Der Innenraum der zweiten Kartusche 52 ist mit einer zweiten pastösen Ausgangskomponente 54 des Mehrkomponenten-PMMA-Knochenzements gefüllt. Der Innenraum der dritten Kartusche 61 ist mit einer dritten pastösen Ausgangskomponente 63 des Mehrkomponenten-PMMA-Knochenzements gefüllt. Die Innenräume der Kartuschen 51, 52, 61 sind auf deren Rückseite (in den Figuren 6 und 8 bis 10 unten und in Figur 7 rechts oben) durch einen ersten Austragskolben 55 in der ersten Kartusche 51, einen zweiten Austragskolben 56 in der zweiten Kartusche 52 und einen dritten Austragskolben 67 in der dritten Kartusche 61 begrenzt, wobei die Austragskolben 55, 56, 67 die Innenräume der Kartuschen 51, 52, 61 nach außen fluiddicht abschließen. Der Austragskolben 55 der ersten Kartusche 51 weist dementsprechend zwei seitliche Ausnehmungen auf, so dass er über die zweite Kartusche 52 und die dritte Kartusche 61 gleiten kann. Der zweite Austragskolben 56 der zweiten Kartusche 52 und der dritte Austragskolben 67 der dritten Kartusche 61 weisen passend zum kleineren Innenraum einen geringeren Durchmesser auf als der Austragskolben 55 der ersten Kartusche 51.

Der Innenraum der zweiten Kartusche 52 und der Innenraum der dritten Kartusche 61 sind zylindrisch mit kreisförmiger Grundfläche geformt. Der Innenraum der ersten Kartusche 51 ist ebenfalls zylindrisch mit kreisförmiger Grundfläche geformt, wobei die zweite Kartusche 52 und die dritte Kartusche 61 einen Teil des Innenraums der ersten Kartusche 51 einnehmen und so eine Brechung der kreisförmig zylindrischen Symmetrie des Innenraums der ersten Kartusche 51 bewirken. Der Austragskolben 55 der ersten Kartusche 51 weist an seiner Rückseite (in den Figuren 6 und 8 bis 10 unten) eine Vertiefung auf, in die eine Pressvorrichtung 57 eingesteckt ist. Die Pressvorrichtung 57 weist an ihrer Rückseite zwei Klemmkanten 58 beziehungsweise eine Klemmkante 58 auf, die in die durch die zweite Kartusche 52 und dritte Kartusche 61 belegten Räume im Innenraum der ersten Kartusche 51 greifen beziehungsweise greift, wenn die Pressvorrichtung 57 innerhalb der ersten Kartusche 51 nach vorne (in den Figuren 6 und 8 bis 10 nach oben) getrieben wird. Die Klemmkanten 58 weisen jeweils eine abgeschrägte Oberfläche auf, die in Richtung senkrecht zur Zylinderachse der zweiten Kartusche 52 und dritten Kartusche 61 geneigt sind. Die zweite Kartusche 52 und die dritte Kartusche 61 haben ein gleich großes Volumen und zusammen ein Volumen von etwa einem Zwanzigstel der ersten Kartusche 51. Dementsprechend wird der Zementteig mit einem Mischungsverhältnis von etwa 40 zu 1 zu 1 aus den drei Ausgangskomponenten 53, 54, 63 gemischt. Aufgrund der zylindrischen Symmetrie der Innenräume der Kartuschen 51, 52, 61 bleibt das Mischungsverhältnis während des Auspressvorgangs konstant.

Anstatt einer dritten Ausgangskomponente 63 kann auch die zweite Komponente 54 sowohl in der zweiten Kartusche 52 als auch in der dritten Kartusche 61 enthalten sein. Dadurch wird ein Zweikomponenten-Knochenzement mit einem Mischungsverhältnis von 20 zu 1 gemischt. Der Vorteil gegenüber dem ersten Ausführungsbeispiel ist darin zu sehen, dass durch den symmetrischen Aufbau während des Austragens der Ausgangskomponenten 51, 52 keine Kräfte senkrecht zur Zylinderachse beziehungsweise Symmetrieachse entstehen können, durch die die Pressvorrichtung 57 verkanten könnte und dadurch in ihrer Bewegung behindert wird.

Die Klemmkante 58 oder die gesamte Pressvorrichtung 57 bestehen aus einem Material und/oder sind derart geformt, dass die Pressvorrichtung 57 oder zumindest die Klemmkante 58 härter oder fester ist als die Wand der zweiten Kartusche 52 und die Wand der dritten Kartusche 61. Bevorzugt besteht die Klemmkante 58 und die gesamte Pressvorrichtung 57 aus einem Metall, insbesondere einer Aluminium-Legierung, oder einem festen Kunststoff, der zumindest härter, fester und/oder zäher ist als das Material der Wand der zweiten inneren Kartusche 52 und der dritten inneren Kartusche 61.

Die Austragskolben 55, 56, 67 sind axial in Längsrichtung in den Innenräumen der Kartuschen 51, 52, 61 in Richtung eines Kartuschenkopfs 59 der Kartuschen 51, 52, 61 beweglich gelagert (in den Figuren 6 und 8 bis 10 von unten nach oben). In dem Kartuschenkopf 59 sind eine Öffnung 72 der ersten Kartusche 51, eine Öffnung 74 der zweiten Kartusche 52 und eine Öffnung 73 der dritten Kartusche 61 vorgesehen (siehe Figur 7). Im Bereich des Kartuschenkopfs 59 ist im Lagerungszustand des Lagerungs- und Mischsystems (siehe Figur 6) eine Überwurfmutter 60 aufgeschraubt, mit der eine gummielastische Platte 71 gehalten wird, die die drei Öffnungen 72, 73, 74 begrenzt und die einen Teil des Kartuschenkopfs 59 bildet. Drei Stopfen 62, 64, 65 sind in die drei Öffnungen 72, 73, 74 eingesteckt, die die Öffnungen 72, 73, 74 und damit die Innenräume der Kartuschen 51, 52, 61 an der Vorderseite (in den Figur 6 oben in Figur 7 unten links, in Richtung des Betrachters) fluiddicht verschließen.

An der Vorderseite der ersten Kartusche 51 ist außen ein Außengewinde 66 als Befestigungselement 66 vorgesehen, auf das die Überwurfmutter 60 aufgeschraubt werden kann. Die Überwurfmutter 60 weist dazu ein passendes Innengewinde 68 als Gegenbefestigungselement 68 auf. An der Rückseite des Lagerungs- und Mischsystems ist ein Stutzen mit einem Anschluss 70 zur Befestigung einer Auspressvorrichtung (nicht gezeigt) vorgesehen. Die Auspressvorrichtung hält die äußere erste Kartusche 51 und weist einen Stößel auf, mit dem die Pressvorrichtung 57 in Richtung des Kartuschenkopfs 59 gedrückt werden kann. Die Auspressvorrichtung wird bevorzugt manuell angetrieben.

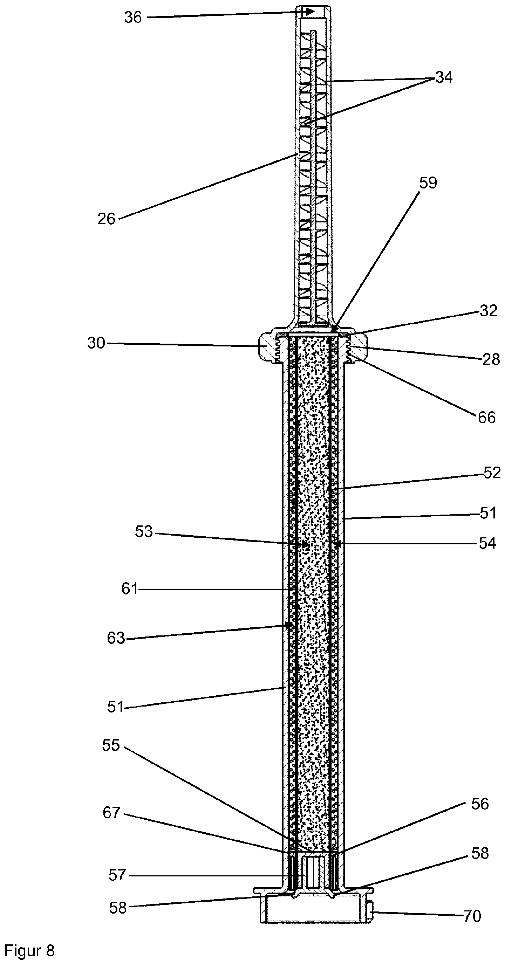

Figur 8 zeigt eine schematische Querschnittansicht durch das zweite erfindungsgemäße Lagerungs- und Mischsystem nach den Figuren 6 und 7 unmittelbar vor der Applikation des PMMA-Knochenzements, bei dem ein Austragsrohr 26 an der Vorderseite der ersten Kartusche 51 befestigt ist. Um das Austragsrohr 26 an der ersten Kartusche 51 zu befestigen, wird die Überwurfmutter 60 abgeschraubt und werden die Stopfen 62, 64, 65 entfernt und so die Kartuschen 51, 52, 61 geöffnet. Anschließend wird auf das Außengewinde 66 das Austragsrohr 26 aufgeschraubt. Dazu ist ein zum Außengewinde 66 passendes Innengewinde 28 an dem Austragsrohr 26 im Bereich eines Stutzens 30 vorgesehen. Das Austragsrohr 26 ist mit einer umlaufenden Dichtung 32 gegen das Lagerungs- und Mischsystem beziehungsweise den Anschluss der ersten Kartusche 51 an dem Kartuschenkopf 59 abgedichtet, damit keine Ausgangskomponenten 53, 54, 63 und kein gemischter Zementteig zwischen dem Austragsrohr 26 und dem Kartuschenkopf 59 nach außen gedrückt wird.

In dem Austragsrohr 26 sind zehn statische Mischelemente 34 angeordnet, die die Ausgangskomponenten 53, 54, 63 miteinander durchmischen, wenn diese durch das Austragsrohr 26 gepresst werden. Dadurch entsteht ein gut durchmischter Zementteig, der über eine Austragsöffnung 36 an der Spitze des Austragsrohrs 26 ausgetragen beziehungsweise appliziert werden kann. Das Austragsrohr 26 kann Teil des zweiten erfindungsgemäßen Lagerungs- und Mischsystems sein. Das Austragsrohr 26 gleicht dem zu dem ersten Ausführungsbeispiel nach den Figuren 1 bis 5 beschriebenen Austragsrohr 26.

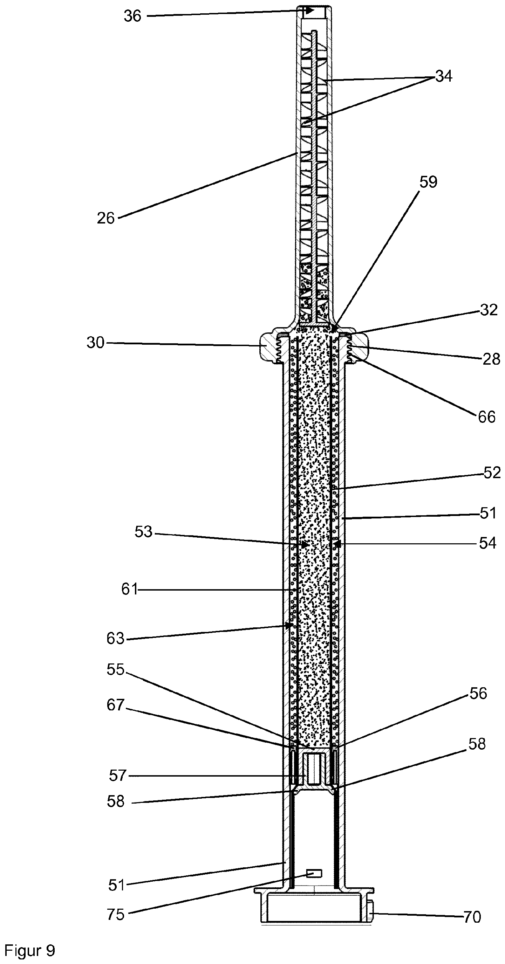

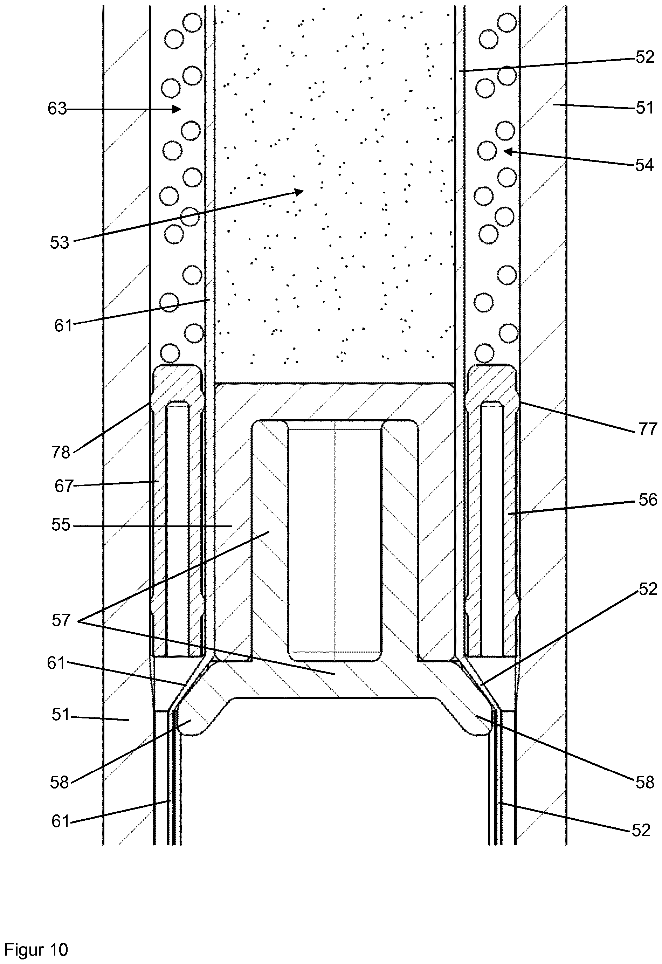

Wenn das Austragsrohr 26 an der ersten Kartusche 51 befestigt ist, wird das Lagerungs- und Mischsystem in die Auspressvorrichtung (nicht gezeigt) eingesetzt und über den Anschluss 70 mit der Auspressvorrichtung verbunden. Der Stößel der Auspressvorrichtung wird bodenseitig in das Lagerungs- und Mischsystem eingetrieben und drückt so bodenseitig auf die Pressvorrichtung 57. Da der erste Austragskolben 55 mit der Pressvorrichtung 57 verbunden ist, wird dieser von der Pressvorrichtung 57 in die erste Kartusche 51 eingeschoben und dabei die erste Komponente 53 aus der ersten Kartusche 51 in das Austragsrohr 26 gedrückt. Gleichzeitig wird die Wand der zweiten Kartusche 52 von der Klemmkante 58 und die Wand der dritten Kartusche 61 von der gegenüberliegenden Klemmkante 58 in Richtung der Innenwand der ersten Kartusche 51 gedrückt. Durch die Verformung der Wand der zweiten Kartusche 52 wird der zweite Austragskolben 56 in Richtung des Kartuschenkopfs 59 gedrückt und so die zweite Ausgangskomponente 54 im Inneren der zweiten Kartusche 52 in das Austragsrohr 26 gepresst. Ebenso wird durch die Verformung der Wand der dritten Kartusche 61 der dritte Austragskolben 67 in Richtung des Kartuschenkopfs 59 gedrückt und so die dritte Ausgangskomponente 63 im Inneren der dritten Kartusche 61 in das Austragsrohr 26 gepresst. Diese Situation ist in den Figuren 9 und 10 dargestellt. Figur 9 zeigt hierzu eine schematische Querschnittansicht durch das zweite erfindungsgemäße Lagerungs- und Mischsystem nach Figur 8 während des Mischvorgangs und Figur 10 eine vergrößerte schematische Querschnittansicht des Antriebs des zweiten erfindungsgemäßen Lagerungs- und Mischsystems nach Figur 9.

An dem ersten Austragskolben 55 sind zwei umlaufende Erhebungen als Dichtungen vorgesehen (in Figur 10 nicht zu sehen, aber analog zur Ausführung nach Figur 5), mit denen der Austragskolben 65 mit den Innenwänden der ersten Kartusche 51 abschließt. Ebenso sind an dem zweiten Austragskolben 56 zwei umlaufende Erhebungen 77 als Dichtungen 77 vorgesehen, mit denen der zweite Austragskolben 56 mit den Innenwänden der zweiten Kartusche 52 abschließt. Ferner sind an dem dritten Austragskolben 67 zwei umlaufende Erhebungen 78 als Dichtungen 78 vorgesehen, mit denen der dritte Austragskolben 67 mit den Innenwänden der dritten Kartusche 61 abschließt. Mit den Dichtungen 77, 78 kann sichergestellt werden, dass der gesamte Inhalt der drei Kartuschen 51, 52, 61, also die drei Ausgangskomponenten 53, 54, 63 restlos ausgetrieben werden und so zum Herstellen einer PMMA-Knochenzement-Mischung mit dem gewünschten Verhältnis genutzt werden können. Dadurch, dass die Wand der zweiten Kartusche 52 erst nach dem Austreiben der zweiten Ausgangskomponente 54 mit dem zweiten Austragskolben 56 zusammengedrückt wird und die Wand der dritten Kartusche 61 erst nach dem Austreiben der dritten Ausgangskomponente 63 (oder alternativ der zweiten Ausgangskomponente 54) mit dem dritten Austragskolben 67 zusammengedrückt wird, können in den beim Zusammendrücken der Wand der zweiten Kartusche 52 und der dritten Kartusche 61 entstehenden Falten keine Reste der zweiten Ausgangskomponente 54 und der dritten Ausgangskomponente 63 zurückbleiben und so das Mischungsverhältnis im Zementteig nicht verfälscht werden.

In der Innenwand der äußeren ersten Kartusche 51 sind zwei gegenüberliegende Vertiefungen 75 als Rastmittel 75 vorgesehen. In dem ersten Austragskolben 55 sind an der äußeren Mantelfläche zwei passende Gegenrastmittel (nicht zu sehen) vorgesehen, die in die Vertiefungen 75 greifen können und den Austragskolben 55 dadurch in der Ausgangsposition halten, die zum Lagern der Ausgangskomponenten 53, 54, 63 geeignet ist (siehe Figur 6). Die Rastung ist durch einen Druck auf die Rückseite des Austragskolbens 55 beziehungsweise auf die Rückseite der Pressvorrichtung 57 lösbar, so dass der erste Austragskolben 55 und damit die Pressvorrichtung 57 in Richtung des Kartuschenkopfs 59 bewegbar ist, wenn der Rastwiderstand überwunden wird.

Mit dem erfindungsgemäßen Aufbau des Lagerungs- und Mischsystems können auch geringe Mengen der zweiten Ausgangskomponente 54 und der dritten Ausgangskomponente 63 im richtigen beziehungsweise gewünschten Mischungsverhältnis beigemischt werden. Herkömmliche Auspressvorrichtungen mit einem zentralen Stößel können dazu verwendet werden, den Zementteig zu mischen und auszutragen, da die Wände der zweiten Kartusche 52 und dritten Kartusche 61 nach außen in Richtung der ersten Kartusche 51 gedrückt wird und so der Bewegung des Stößels nicht im Wege sind.

Die äußere erste Kartusche 51 kann dabei derart schmal gestaltet werden, erfindungsgemäß bevorzugt mit einem Innendurchmesser von maximal 25 mm oder besonders bevorzugt mit einem Innendurchmesser von maximal 20 mm, dass die zähen Ausgangskomponenten 53, 54, 63 insbesondere die zähe erste Ausgangskomponente 53, in das Austragsrohr 26 und durch die statischen Mischer 34 gedrückt werden kann, ohne dass der Widerstand der zähen Pasten 53, 54, 63 derart groß würde, dass diese nicht mehr mit herkömmlichen, manuell angetriebenen Auspressvorrichtungen ausgetrieben werden könnten.

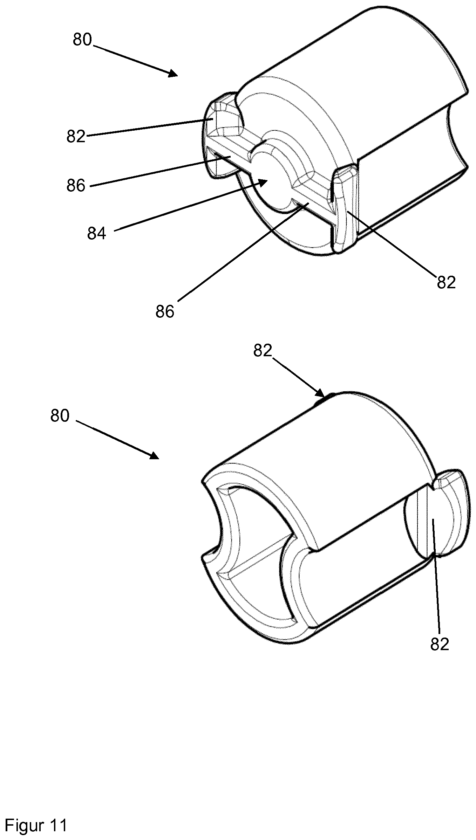

Figur 11 zeigt zwei schematische perspektivische Ansichten einer Pressvorrichtung 80 für ein erfindungsgemäßes Lagerungs- und Mischsystem mit zwei inneren Kartuschen und zwar auf die Rückseite (oben) und auf die Vorderseite (unten). Die Pressvorrichtung 80 weist zwei abgeschrägte Klemmkanten 82 auf, mit denen die Wände einer zweiten und einer dritten Kartusche im Inneren einer äußeren ersten Kartusche seitlich weggedrückt werden können (analog dem zweiten Ausführungsbeispiel nach den Figuren 6 bis 10). Auf der Rückseite der Pressvorrichtung 80 befindet sich eine Pressfläche 84, auf die sich der Stößel einer Auspressvorrichtung (nicht gezeigt) abstützen kann, um die Pressvorrichtung 80 in eine Kartusche einzudrücken. Ferner ist an der Rückseite der Pressvorrichtung 80 ein Steg 86 vorgesehen, der die beiden bodenseitigen Enden der Klemmkanten 82 und die vorstehende Pressfläche 84 verbindet und dadurch für eine mechanische Stabilisierung der Form der Pressvorrichtung 80 sorgt. Mit dieser Ausführung wird die Position und Lage der Klemmkanten 82 stabilisiert. Dadurch können die Wände der zweiten und dritten Kartusche leichter verformt werden.

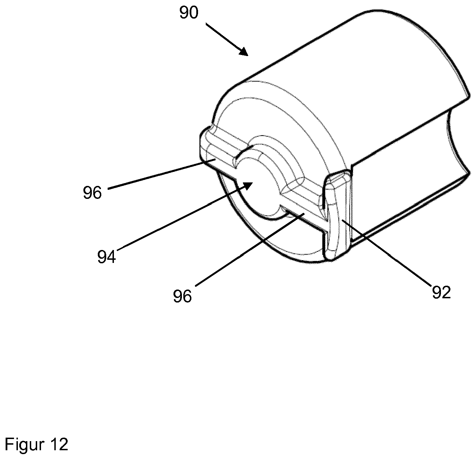

Figur 12 zeigt eine schematische perspektivische Ansicht einer Pressvorrichtung 90 für ein erfindungsgemäßes Lagerungs- und Mischsystem mit einer inneren Kartusche, wie das nach den Figuren 1 bis 5 gezeigte erste Ausführungsbeispiel. Die Pressvorrichtung 90 weist eine abgeschrägte Klemmkante 92 auf, mit denen die Wände einer zweiten Kartusche im Inneren einer äußeren ersten Kartusche seitlich weggedrückt werden können (analog dem ersten Ausführungsbeispiel nach den Figuren 1 bis 5). Auf der Rückseite der Pressvorrichtung 90 befindet sich eine Pressfläche 94, auf die sich der Stößel einer Auspressvorrichtung (nicht gezeigt) abstützen kann, um die Pressvorrichtung 90 in eine Kartusche einzudrücken. Ferner ist an der Rückseite der Pressvorrichtung 90 ein Steg 96 vorgesehen, der das bodenseitige Ende der Klemmkante 92 und die vorstehende Pressfläche 94 verbindet und dadurch für eine mechanische Stabilisierung der Form der Pressvorrichtung 90 sorgt. Mit dieser Ausführung wird die Position und Lage der Klemmkante 92 stabilisiert. Dadurch können die Wände der zweiten Kartusche leichter verformt werden.

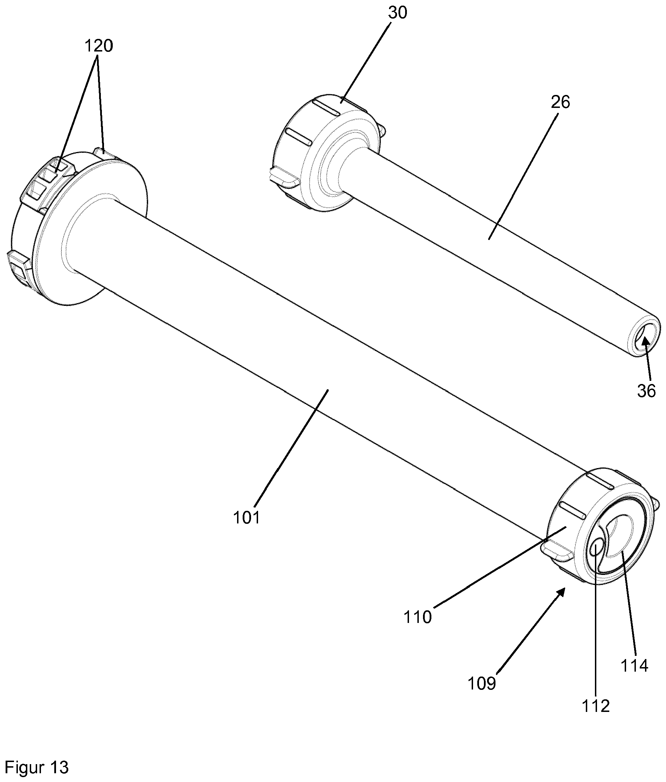

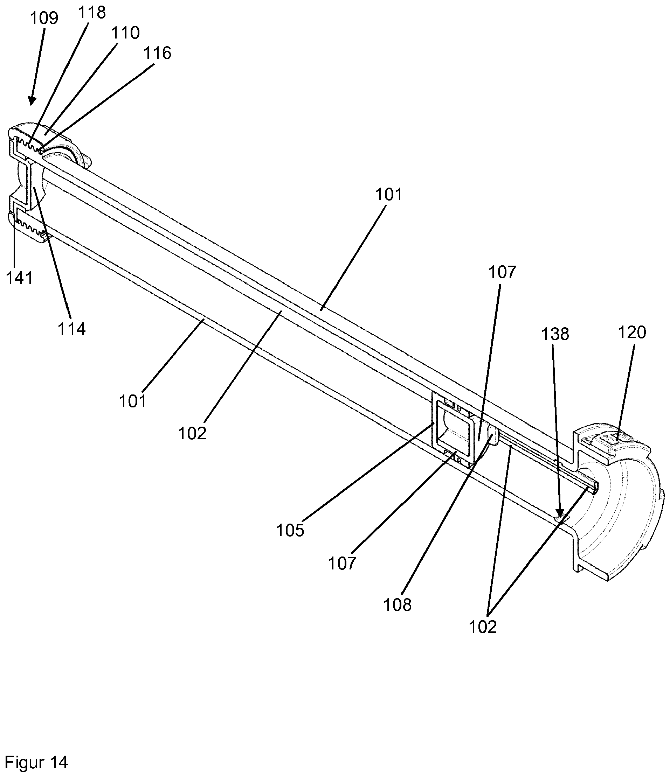

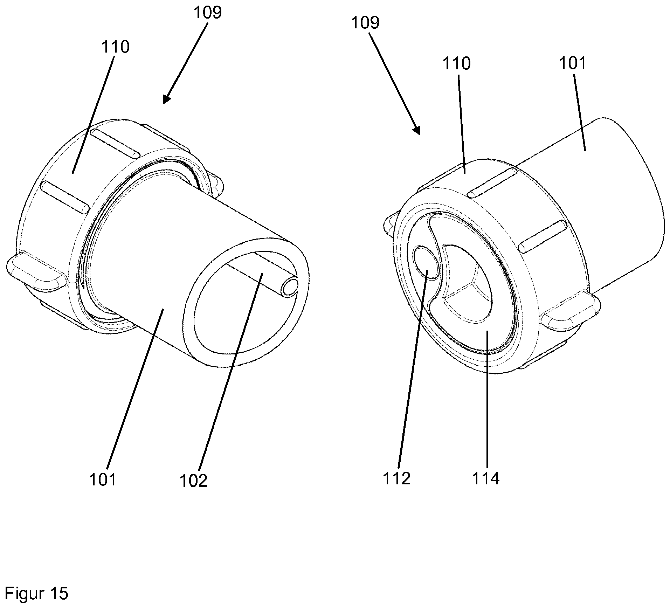

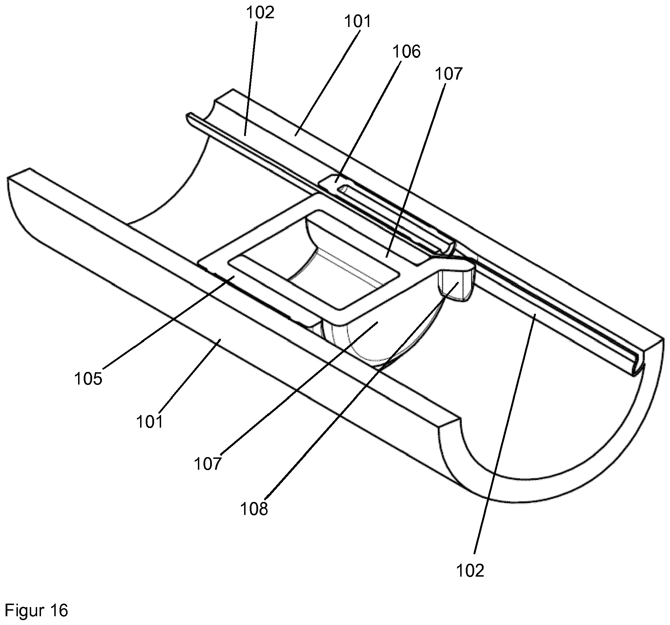

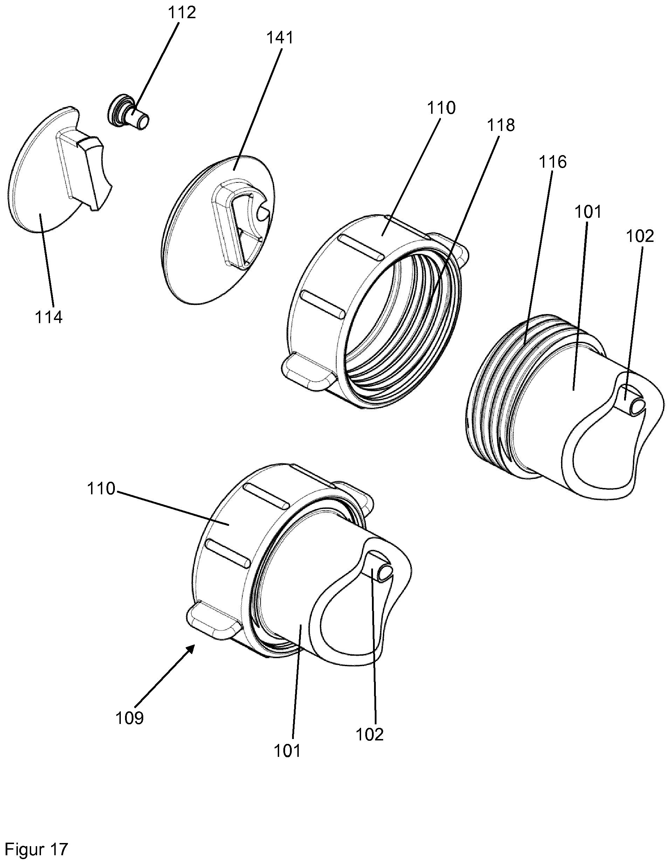

Die Figuren 13 bis 17 zeigen eine dritte beispielhafte Ausführung eines erfindungsgemäßen Lagerungs- und Mischsystems. Dabei zeigt Figur 13 eine schematische perspektivische Ansicht auf das dritte, alternative erfindungsgemäße Lagerungs- und Mischsystem und Figur 14 eine schematische perspektivische Querschnittansicht des dritten erfindungsgemäßen Lagerungs- und Mischsystems nach Figur 13 mit eingeschobener Pressvorrichtung 107. Figur 15 zeigt zwei schematische perspektivische Schnittansichten des vorderen Bereichs des dritten erfindungsgemäßen Lagerungs- und Mischsystems nach den Figuren 13 und 14, Figur 16 eine Vergrößerung eines Teils des dritten erfindungsgemäßen Lagerungs- und Mischsystems nach den Figuren 13 bis 15 als schematische perspektivische Querschnittansicht und Figur 17 eine Explosionsdarstellung (oben) und eine Schnittansicht (unten) des vorderen Bereichs des dritten erfindungsgemäßen Lagerungs- und Mischsystems nach Figur 15.

Analog zum ersten Ausführungsbeispiel nach den Figuren 1 bis 5 weist das dritte alternative Lagerungs- und Mischsystem nach den Figuren 13 bis 17 eine äußere erste Kartusche 101 auf, in der eine innere zweite Kartusche 102 an der Innenwand der ersten Kartusche 101 über die gesamte Länge der ersten Kartusche 101 befestigt ist. Beide Kartuschen 101, 102 sind aus dem gleichen Material gefertigt. Die Wandstärke der inneren zweiten Kartusche 102 entspricht etwa einem Viertel der Wandstärke der äußeren ersten Kartusche 101. Der Innenraum der ersten Kartusche 101 ist bis auf den Raum, den die zweite Kartusche 102 einnimmt, mit einer ersten pastösen Ausgangskomponente (nicht gezeigt) eines PMMA-Knochenzements gefüllt oder kann mit einer solchen ersten pastösen Ausgangskomponente gefüllt werden. Der Innenraum der zweiten Kartusche 102 ist mit einer zweiten pastösen Ausgangskomponente (nicht gezeigt) des Zweikomponenten-PMMA-Knochenzements gefüllt oder kann mit einer solchen zweiten pastösen Ausgangskomponente gefüllt werden. Die Ausgangskomponenten müssen also nicht in dem Lagerungs- und Mischsystem enthalten sein.

Die Innenräume der Kartuschen 101, 102 sind auf deren Rückseite (in Figur 13 links oben, in Figur 14 rechts unten) durch einen ersten Austragskolben 105 in der ersten Kartusche 101 und einen zweiten Austragskolben 106 in der zweiten Kartusche 102 begrenzt, wobei die Austragskolben 105, 106 die Innenräume der Kartuschen 101, 102 nach außen fluiddicht abschließen. Der Austragskolben 105 der ersten Kartusche 101 weist dementsprechend eine seitliche Ausnehmung auf, so dass er über die zweite Kartusche 102 gleiten kann. Der Austragskolben 106 der zweiten Kartusche 102 weist passend zum kleineren Innenraum einen geringeren Durchmesser auf als der Austragskolben 105 der ersten Kartusche 101.

Der Innenraum der zweiten Kartusche 102 ist zylindrisch mit kreisförmiger Grundfläche geformt. Der Innenraum der ersten Kartusche 101 ist ebenfalls zylindrisch mit kreisförmiger Grundfläche geformt, wobei die zweite Kartusche 102 einen Teil des Innenraums der ersten Kartusche 101 einnimmt und so eine Brechung der kreisförmig zylindrischen Symmetrie des Innenraums der ersten Kartusche 101 bewirkt. Der Austragskolben 105 der ersten Kartusche 101 weist an seiner Rückseite (in Figur 13 links oben, in Figur 14 rechts unten) eine Vertiefung auf, in die eine Pressvorrichtung 107 eingesteckt ist. Die Pressvorrichtung 107 weist an ihrer Rückseite eine Klemmkante 108 auf, die in den durch die zweite Kartusche 102 belegten Raum im Innenraum der ersten Kartusche 101 greift, wenn die Pressvorrichtung 107 innerhalb der ersten Kartusche 101 nach vorne (in den Figuren 14, 16 und 17 nach links oben, in Figur 13 nach rechts unten) getrieben wird. Die Klemmkante 108 weist eine abgeschrägte Oberfläche auf, die in Richtung senkrecht zur Zylinderachse der zweiten Kartusche 102 geneigt ist. Die zweite Kartusche 102 hat ein Volumen von etwa einem Zwanzigstel der ersten Kartusche 101. Dementsprechend wird der Zementteig mit einem Mischungsverhältnis von etwa 20 zu 1 aus den beiden Ausgangskomponenten gemischt. Aufgrund der zylindrischen Symmetrie der Innenräume der Kartuschen 101, 102 bleibt das Mischungsverhältnis während des Auspressvorgangs konstant.

Die Klemmkante 108 oder die gesamte Pressvorrichtung 107 bestehen aus einem Material und/oder sind derart geformt, dass die Pressvorrichtung 107 oder zumindest die Klemmkante 108 härter oder fester ist als die Wand der zweiten Kartusche 102. Bevorzugt besteht die Klemmkante 108 und die gesamte Pressvorrichtung 107 aus einem Metall, insbesondere einer Aluminium-Legierung, oder einem festen Kunststoff, der zumindest härter, fester und/oder zäher ist als das Material der Wand der zweiten inneren Kartusche 102, beispielsweise einem faserverstärkten Kunststoff.

Die Austragskolben 105, 106 sind axial in Längsrichtung in den Innenräumen der Kartuschen 101, 102 in Richtung eines Kartuschenkopfs 109 der Kartuschen 101, 102 beweglich gelagert (in den Figuren 14, 16 und 17 nach links oben vom Betrachter weg und in Figur 13 nach rechts unten zum Betrachter hin). In dem Kartuschenkopf 109 sind eine Öffnung der ersten Kartusche 1 und eine Öffnung der zweiten Kartusche 102 vorgesehen. Im Bereich des Kartuschenkopfs 109 ist im Lagerungszustand des Lagerungs- und Mischsystems (siehe Figur 13) eine Überwurfmutter 110 aufgeschraubt, mit der eine gummielastische Platte 141 gehalten wird, die die beiden Öffnungen begrenzt und die einen Teil des Kartuschenkopfs 109 bildet. Zwei Stopfen 112, 114 sind in die Öffnungen eingesteckt, die die Öffnungen und damit die Innenräume der Kartuschen 101, 102 an der Vorderseite fluiddicht verschließen.

An der Vorderseite der ersten Kartusche 101 ist außen ein Außengewinde 116 als Befestigungselement 116 vorgesehen, auf das die Überwurfmutter 110 aufgeschraubt werden kann. Die Überwurfmutter 110 weist dazu ein passendes Innengewinde 118 als Gegenbefestigungselement 118 auf. An der Rückseite des Lagerungs- und Mischsystems ist ein Stutzen mit einem Anschluss 120 zur Befestigung einer Auspressvorrichtung (nicht gezeigt) vorgesehen. Die Auspressvorrichtung hält die äußere erste Kartusche 101 und weist einen Stößel auf, mit dem die Pressvorrichtung 107 in Richtung des Kartuschenkopfs 109 gedrückt werden kann. Die Auspressvorrichtung wird bevorzugt manuell angetrieben.

Das Austragsrohr 26 kann analog dem ersten Ausführungsbeispiel an der Vorderseite der ersten Kartusche 101 befestigt werden und ist analog dem ersten Ausführungsbeispiel nach den Figuren 1 bis 5 aufgebaut. Um das Austragsrohr 26 an der ersten Kartusche 101 zu befestigen, wird die Überwurfmutter 110 abgeschraubt und werden die Stopfen 112, 114 entfernt und so die Kartuschen 101, 102 geöffnet. Anschließend wird auf das Außengewinde 116 das Austragsrohr 26 aufgeschraubt. Das Austragsrohr 26 kann Teil des erfindungsgemäßen Lagerungs- und Mischsystems sein.

Wenn das Austragsrohr 26 an der ersten Kartusche 101 befestigt ist, wird das Lagerungs- und Mischsystem in die Auspressvorrichtung (nicht gezeigt) eingesetzt und über den Anschluss 120 mit der Auspressvorrichtung verbunden. Der Stößel der Auspressvorrichtung wird bodenseitig in das Lagerungs- und Mischsystem eingetrieben und drückt so bodenseitig auf die Pressvorrichtung 107. Da der erste Austragskolben 105 mit der Pressvorrichtung 107 verbunden ist, wird dieser von der Pressvorrichtung 107 in die erste Kartusche 101 eingeschoben und dabei die erste Komponente 103 aus der ersten Kartusche 101 in das Austragsrohr 26 gedrückt. Gleichzeitig wird die Wand der zweiten Kartusche 102 von der Klemmkante 108 in Richtung der Innenwand der ersten Kartusche 101 gedrückt. Durch die Verformung der Wand der zweiten Kartusche 102 wird der zweite Austragskolben 106 in Richtung des Kartuschenkopfs 109 gedrückt und so die zweite Ausgangskomponente 104 im Inneren der zweiten Kartusche 102 in das Austragsrohr 26 gepresst.

Dadurch, dass die Wand der zweiten Kartusche 102 erst nach dem Austreiben der zweiten Ausgangskomponente 104 mit dem zweiten Austragskolben 106 beziehungsweise hinter dem zweiten Austragskolben 106 zusammengedrückt wird, können in den beim Zusammendrücken der Wand der zweiten Kartusche 102 entstehenden Falten keine Reste der zweiten Ausgangskomponente 104 zurückbleiben und so das Mischungsverhältnis im Zementteig nicht verfälscht werden.

Mit dem erfindungsgemäßen Aufbau des Lagerungs- und Mischsystems können auch geringe Mengen der zweiten Ausgangskomponente 104 im richtigen beziehungsweise gewünschten Mischungsverhältnis beigemischt werden. Herkömmliche Auspressvorrichtungen mit einem zentralen Stößel können dazu verwendet werden, den Zementteig zu mischen und auszutragen, da die Wand der zweiten Kartusche 102 nach außen in Richtung der ersten Kartusche 101 gedrückt wird und so der Bewegung des Stößels nicht im Wege ist.

Die äußere erste Kartusche 101 kann dabei derart schmal gestaltet werden, erfindungsgemäß bevorzugt mit einem Innendurchmesser von maximal 25 mm oder besonders bevorzugt mit einem Innendurchmesser von maximal 20 mm, dass die zähen Ausgangskomponenten 103, 104, insbesondere die zähe erste Ausgangskomponente 103, in das Austragsrohr 26 und durch die statischen Mischer 34 im Austragsrohr 26 gedrückt werden kann, ohne dass der Widerstand der zähen Pasten 103, 104 derart groß würde, dass diese nicht mehr mit herkömmlichen, manuell angetriebenen Auspressvorrichtungen ausgetrieben werden könnten.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 51, 101: Äußere erste Kartusche
- 2, 52, 102: Innere zweite Kartusche
- 3, 53: Erste Ausgangskomponente
- 4, 54: Zweite Ausgangskomponente
- 5, 55, 105: Erster Austragskolben
- 6, 56, 106: Zweiter Austragskolben
- 7, 57, 107: Pressvorrichtung
- 8, 58, 108: Klemmkante
- 9, 59, 109: Kartuschenkopf
- 10, 60, 110: Überwurfmutter
- 12, 62, 112: Stopfen
- 14, 64, 114: Stopfen
- 16, 66, 116: Außengewinde / Befestigungselement
- 18, 68, 118: Innengewinde / Befestigungselement
- 20, 70, 120: Anschluss
- 22, 72: Öffnung der ersten Kartusche
- 24, 74: Öffnung der zweiten Kartusche
- 26: Austragsrohr
- 28: Innengewinde
- 30: Stutzen
- 32: Dichtung
- 34: Statischer Mischer
- 36: Austragsöffnung
- 38, 75, 138: Rastmittel / Vertiefung
- 40: Dichtung / Erhebung
- 41, 71, 141: Gummielastische Platte
- 42, 77: Dichtung / Erhebung
- 61: Innere dritte Kartusche
- 63: Dritte Ausgangskomponente
- 65: Stopfen
- 67: Dritter Austragskolben
- 73: Öffnung der dritten Kartusche
- 78: Dichtung / Erhebung
- 80, 90: Pressvorrichtung
- 82, 92: Klemmkante
- 84, 94: Pressfläche für Stößel
- 86, 96: Steg

## Patentansprüche

1. Lagerungs- und Mischsystem für pastenförmige Mehrkomponenten-Polymethylmethacrylat-Knochenzemente, das Lagerungs- und Mischsystem aufweisend
eine erste röhrenförmige Kartusche (1, 51, 101) mit einem ersten zylindrischen Innenraum, wobei in dem Innenraum eine erste Ausgangskomponente (3, 53) eines Mehrkomponenten-Knochenzements enthalten ist,
einen ersten Austragskolben (5, 55, 105) der axial beweglich im ersten Innenraum der ersten Kartusche (1, 51, 101) angeordnet ist und der zum Austreiben der ersten Ausgangskomponente (3, 53) aus der ersten Kartusche (1, 51, 101) durch eine dem ersten Austragskolben (5, 55, 105) gegenüberliegende Öffnung (22, 72) in einem Kartuschenkopf (9, 59, 109) der ersten Kartusche (1, 51, 101) vorgesehen ist,
eine zweite röhrenförmige Kartusche (2, 52, 102), die innerhalb der ersten röhrenförmigen Kartusche (1, 51, 101) angeordnet ist, wobei die zweite Kartusche (2, 52, 102) mit ihrer Außenwand an der Innenwand der ersten Kartusche (1, 51, 101) anliegt und an der Innenwand der ersten Kartusche (1, 51, 101) befestigt ist, wobei in der zweiten Kartusche (2, 52, 102) eine zweite Ausgangskomponente (4, 54) des Mehrkomponenten-Knochenzements enthalten ist und ein zweiter Austragskolben (6, 56, 106) angeordnet ist, wobei mit dem zweiten Austragskolben (6, 56, 106) die zweite Ausgangskomponente (4, 54) aus der zweiten Kartusche (2, 52, 102) durch eine gegenüberliegende Öffnung (24, 74) in der zweiten Kartusche (2, 52, 102) im Bereich des Kartuschenkopfs (9, 59, 109) der ersten Kartusche (1, 51, 101) auszutreiben ist,
wobei vom Kartuschenkopf (9, 59, 109) aus gesehen hinter dem ersten Austragskolben (5, 55, 105) und dem zweiten Austragskolben (6, 56, 106) eine axial im Innenraum der ersten Kartusche (1, 51, 101) vortreibbare Pressvorrichtung (7, 57, 80, 90, 107) mit einer Klemmkante (8, 58, 82, 92, 108) zum Zusammenpressen der zweiten Kartusche (2, 52, 102) angeordnet ist, wobei die Pressvorrichtung (7, 57, 80, 90, 107) derart in Richtung des Kartuschenkopfs (9, 59, 109) vortreibbar ist, dass während der Bewegung der Pressvorrichtung (7, 57, 80, 90, 107) die zweite Kartusche (2, 52, 102) axial fortlaufend zusammengepresst wird und dabei der erste Austragskolben (5, 55, 105) und der zweite Austragskolben (6, 56, 106) in Richtung des Kartuschenkopfs (9, 59, 109) vorgetrieben werden.

2. Lagerungs- und Mischsystem nach Anspruch 1, **dadurch gekennzeichnet, dass**
die zweite Kartusche (2, 52, 102) mit ihrer Außenwand mit der Innenwand der ersten Kartusche (1, 51, 101) vorne im Bereich des Kartuschenkopfs (9, 59, 109) und hinten, hinter dem zweiten Austragskolben (6, 56, 106) befestigt ist, wobei vorzugsweise die zweite Kartusche (2, 52, 102) mit ihrer Außenwand mit der Innenwand der ersten Kartusche (1, 51, 101) entlang der gesamten Länge der zweiten Kartusche (2, 52, 102) befestigt ist.

3. Lagerungs- und Mischsystem nach Anspruch 1 oder 2, **dadurch gekennzeichnet, dass**
die Öffnungen (22, 24, 72, 74) mit einem lösbaren Verschluss (12, 14, 62, 64, 112, 114) an dem Kartuschenkopf (9, 59, 109) verschlossen sind.

4. Lagerungs- und Mischsystem nach einem der vorangehenden Ansprüche, **dadurch gekennzeichnet, dass**
beim Vortreiben der Pressvorrichtung (7, 57, 80, 90, 107) der erste Austragskolben (5, 55, 105) und der zweite Austragskolben (6, 56, 106) parallel zueinander vorgetrieben werden, vorzugsweise der erste Austragskolben (5, 55, 105) und der zweite Austragskolben (6, 56, 106) auf gleicher Höhe in Richtung des Kartuschenkopfs (9, 59, 109) laufen.

5. Lagerungs- und Mischsystem nach einem der vorangehenden Ansprüche, **dadurch gekennzeichnet, dass**
im Bereich des Kartuschenkopfs (9, 59, 109) außen an der ersten Kartusche (1, 51, 101) ein Befestigungsmittel (16, 66, 116), insbesondere ein Außengewinde (16, 66, 116), vorgesehen ist.

6. Lagerungs- und Mischsystem nach einem der vorangehenden Ansprüche, **dadurch gekennzeichnet, dass**
das Lagerungs- und Mischsystem ein Austragsrohr (26) mit einem statischen Mischer (34) aufweist, das an der ersten Kartusche (1, 51, 101) zu befestigen ist, vorzugsweise an dem Befestigungsmittel (16, 66, 116) an der ersten Kartusche (1, 51, 101), zu befestigen ist, wobei besonders bevorzugt an dem Austragsrohr (26) ein zum Außengewinde (16, 66, 116) an der ersten Kartusche (1, 51, 101) passendes Innengewinde (28) vorgesehen ist und/oder Elemente eines Bajonettverschlusses und/oder Rastelemente eines Rastverschlusses vorgesehen sind.

7. Lagerungs- und Mischsystem nach Anspruch 6, **dadurch gekennzeichnet, dass**
das Verhältnis vom Innendurchmesser der ersten Kartusche (1, 51, 101) zum Innendurchmesser des Austragsrohrs (26) kleiner 5 zu 2 ist, wobei bevorzugt das Verhältnis vom Innendurchmesser der ersten Kartusche (1, 51, 101) zum Innendurchmesser des Austragsrohr (26) bevorzugt kleiner oder gleich 2 zu 1 ist und ganz besonders bevorzugt das Verhältnis vom Innendurchmesser der ersten Kartusche (1, 51, 101) zum Innendurchmesser des Austragsrohrs (26) 8 zu 5 ist.

8. Lagerungs- und Mischsystem nach einem der vorangehenden Ansprüche, **dadurch gekennzeichnet, dass**
die Klemmkante (8, 58, 82, 92, 108) gegen die Längsachse der ersten Kartusche (1, 51, 101) geneigt ist, vorzugsweise mit einem Winkel von wenigstens 40° senkrecht zur Längsachse in Richtung der Innenwand der ersten Kartusche (1, 51, 101) geneigt ist, besonders bevorzugt mit einem Winkel zwischen 40° und 80° senkrecht zur Längsachse in Richtung der Innenwand der ersten Kartusche (1, 51, 101) geneigt ist.

9. Lagerungs- und Mischsystem nach einem der vorangehenden Ansprüche, **dadurch gekennzeichnet, dass**
die Klemmkante (8, 58, 82, 92, 108) beim Vortreiben der Pressvorrichtung (7, 57, 80, 90, 107) die zweite Kartusche (2, 52, 102) gegen die Innenwand der ersten Kartusche (1, 51, 101) quetscht.

10. Lagerungs- und Mischsystem nach einem der vorangehenden Ansprüche, **dadurch gekennzeichnet, dass**
die Klemmkante (8, 58, 82, 92, 108) mindestens 30 Prozent der Fläche des Querschnitts der zweiten Kartusche (2, 52, 102) überdeckt, bevorzugt mindestens 60% der Fläche des Querschnitts der zweiten Kartusche (2, 52, 102) überdeckt.

11. Lagerungs- und Mischsystem nach einem der vorangehenden Ansprüche, **dadurch gekennzeichnet, dass**
zwischen der Pressvorrichtung (7, 57, 80, 90, 107) und der Innenwand der ersten Kartusche (1, 51, 101) im Bereich der zweiten Kartusche (2, 52, 102) ein Spalt vorgesehen ist, der genauso breit oder breiter ist als die Dicke der Wand der zweiten Kartusche (2, 52, 102).

12. Lagerungs- und Mischsystem nach einem der vorangehenden Ansprüche, **dadurch gekennzeichnet, dass**
die Rückseite der Pressvorrichtung (7, 57, 80, 90, 107) als Auflagefläche (84, 94) für einen Stößel einer Auspressvorrichtung ausgebildet ist.

13. Lagerungs- und Mischsystem nach einem der vorangehenden Ansprüche, **dadurch gekennzeichnet, dass**
der Durchmesser des Innenraums der ersten Kartusche (1, 51, 101) kleiner oder gleich 25 mm ist, wobei bevorzugt der Durchmesser des Innenraums der ersten Kartusche (1, 51, 101) kleiner oder gleich 20 mm ist, und/oder die erste Kartusche (1, 51, 101) einen Innendurchmesser von höchstens 25 mm und die zweite Kartusche (2, 52, 102) einen Innendurchmesser von höchstens 5 mm haben, bevorzugt die erste Kartusche (1, 51, 101) einen Innendurchmesser von höchstens 20 mm und die zweite Kartusche (2, 52, 102) einen Innendurchmesser von höchstens 3 mm haben.

14. Lagerungs- und Mischsystem nach einem der vorangehenden Ansprüche, **dadurch gekennzeichnet, dass**
beim Vortreiben der Pressvorrichtung (7, 57, 80, 90, 107) in Richtung Kartuschenkopf (9, 59, 109) die Klemmkante (8, 58, 82, 92, 108) durch Quetschung der zweiten Kartusche (2, 52, 102) die so deformierte Wand der zweiten Kartusche (2, 52, 102) gegen die Unterseite des zweiten Austragskolbens (6, 56, 106) gepresst wird und so den zweiten Austragskolben (6, 56, 106) in Richtung Kartuschenkopf (9, 59, 109) schiebt.

15. Lagerungs- und Mischsystem nach einem der vorangehenden Ansprüche, **dadurch gekennzeichnet, dass**
die erste Kartusche (1, 51, 101), die zweite Kartusche (2, 52, 102), der Kartuschenkopf (9, 59, 109) und die Austragskolben (5, 6, 55, 56, 63, 105, 106) aus Kunststoff gefertigt sind, wobei als Kunststoffe Polyethylen-covinylalkohol (EVOH), Polybutylenterephthalat (PBT), Polyethylenterephthalat (PET) und Poly-methacrylsäuremethylester-co-acrylnitril bevorzugt werden.

16. Lagerungs- und Mischsystem nach einem der vorangehenden Ansprüche, **dadurch gekennzeichnet, dass**
das Verhältnis des Volumens der ersten Kartusche (1, 51, 101) zum Volumen der zweiten Kartusche (2, 52, 102) mindestens 95 zu 5 ist, bevorzugt mindestens 98 zu 2 ist.

17. Lagerungs- und Mischsystem nach einem der vorangehenden Ansprüche, **dadurch gekennzeichnet, dass**
die zweite Kartusche (2, 52, 102) bei einer Beaufschlagung der Pressvorrichtung (7, 57, 80, 90, 107) von mindestens 0,5 kN in Richtung des Kartuschenkopfs (9, 59, 109) so gequetscht wird, dass die gequetschte zweite Kartusche (2, 52, 102) durch einen Spalt zwischen der Klemmkante (8, 58, 82, 92, 108) und der Innenwand der ersten Kartusche (1, 51, 101) passt.

18. Lagerungs- und Mischsystem nach einem der vorangehenden Ansprüche, **dadurch gekennzeichnet, dass**
das Verhältnis der Dicke der Wand der ersten Kartusche (1, 51, 101) zu der Dicke der Wand der zweiten Kartusche (2, 52, 102) mindestens 11 zu 10 ist, und wobei bevorzugt das Verhältnis der Dicke der Wand der ersten Kartusche (1, 51, 101) zu der Dicke der Wand der zweiten Kartusche (2, 52, 102) mindestens 2 zu 1 ist.

19. Lagerungs- und Mischsystem nach einem der vorangehenden Ansprüche, **dadurch gekennzeichnet, dass**
ein Teil der Wand der ersten Kartusche (1, 51, 101) einen Teil der Innenwand der zweiten Kartusche (2, 52, 102) bildet, bevorzugt über die gesamte Länge der zweiten Kartusche (2, 52, 102) oder über zumindest 80% der gesamten Länge der zweiten Kartusche (2, 52, 102), und/oder dass ein Teil der Wand der ersten Kartusche (1, 51, 101) einen Teil des zweiten Innenraums des zweiten Kartusche (2, 52, 102) begrenzt, bevorzugt über die gesamte Länge der zweiten Kartusche (2, 52, 102) oder über zumindest 80% der gesamten Länge der zweiten Kartusche (2, 52, 102).

20. Lagerungs- und Mischsystem nach einem der vorangehenden Ansprüche, **dadurch gekennzeichnet, dass**
eine dritte röhrenförmige Kartusche (61) innerhalb der ersten röhrenförmigen Kartusche (1, 51, 101) angeordnet ist, wobei die dritte Kartusche (61) mit ihrer Außenwand an der Innenwand der ersten Kartusche (1, 51, 101) anliegt und an der Innenwand der ersten Kartusche (1, 51, 101) befestigt ist, wobei in der dritten Kartusche (61) die zweite Ausgangskomponente (4, 54) oder eine dritte Ausgangskomponente (63) des Mehrkomponenten-Knochenzements enthalten ist und ein dritter Austragskolben (67) angeordnet ist, wobei mit dem dritten Austragskolben (67) die zweite Ausgangskomponente (4, 54) oder die dritte Ausgangskomponente (63) aus der dritten Kartusche (61) durch eine gegenüberliegende Öffnung (73) in der dritten Kartusche (61) im Bereich des Kartuschenkopfs (9, 59, 109) der ersten Kartusche (1, 51, 101) auszutreiben ist, wobei
die Pressvorrichtung (7, 57, 80, 90, 107) vom Kartuschenkopf (9, 59, 109) aus gesehen hinter dem dritten Austragskolben (67) angeordnet ist und die Pressvorrichtung (7, 57, 80, 90, 107) eine Klemmkante (8, 58, 82, 92, 108) zum Zusammenpressen der dritten Kartusche (61) aufweist, wobei die Pressvorrichtung (7, 57, 80, 90, 107) derart in Richtung des Kartuschenkopfs (9, 59, 109) vortreibbar ist, dass während der Bewegung der Pressvorrichtung (7, 57, 80, 90, 107) die dritte Kartusche (61) axial fortlaufend zusammengepresst wird und dabei der erste Austragskolben (5, 55, 105), der zweite Austragskolben (6, 56, 106) und der dritte Austragskolben (67) in Richtung des Kartuschenkopfs (9, 59, 109) vorgetrieben werden.

21. Lagerungs- und Mischsystem nach Anspruch 20, **dadurch gekennzeichnet, dass**
die dritte Kartusche (61) und/oder der dritte Austragskolben (67) die gleichen Merkmale wie die zweite Kartusche (2, 52, 102) und/oder der zweite Austragskolben (6, 56, 106) nach einem der Ansprüche 2 bis 19 aufweist.

22. Lagerungs- und Mischsystem nach einem der Ansprüche 20 oder 21, **dadurch gekennzeichnet, dass**
zumindest eine vierte röhrenförmige Kartusche innerhalb der ersten röhrenförmigen Kartusche (1, 51, 101) angeordnet ist, wobei die zumindest eine vierte Kartusche mit ihrer Außenwand an der Innenwand der ersten Kartusche (1, 51, 101) anliegt und an der Innenwand der ersten Kartusche (1, 51, 101) befestigt ist, wobei in der zumindest einen vierten Kartusche die zweite, die dritte, eine vierte und/oder jeweils zumindest eine weitere Ausgangskomponente des Knochenzements enthalten ist und in der zumindest einen vierten Kartusche jeweils ein vierter Austragskolben angeordnet ist, wobei mit dem vierten Austragskolben die zweite, die dritte, die vierte und/oder die jeweilige weitere Ausgangskomponente aus der zumindest einen vierten Kartusche durch eine gegenüberliegende Öffnung in der zumindest einen vierten Kartusche im Bereich des Kartuschenkopfs (9, 59, 109) der ersten Kartusche (1, 51, 101) auszutreiben ist, wobei die Pressvorrichtung (7, 57, 80, 90, 107) vom Kartuschenkopf (9, 59, 109) aus gesehen hinter dem oder den vierten Austragskolben angeordnet ist und die Pressvorrichtung (7, 57, 80, 90, 107) zumindest eine Klemmkante (8, 58, 82, 92, 108) zum Zusammenpressen der zumindest einen vierten Kartusche aufweist, wobei die Pressvorrichtung (7, 57, 80, 90, 107) derart in Richtung des Kartuschenkopfs (9, 59, 109) vortreibbar ist, dass während der Bewegung der Pressvorrichtung (7, 57, 80, 90, 107) die zumindest eine vierte Kartusche axial fortlaufend zusammengepresst wird und dabei der erste Austragskolben (5, 55, 105), der zweite Austragskolben (6, 56, 106), der dritte Austragskolben (67) und der vierte oder die vierten Austragskolben in Richtung des Kartuschenkopfs (9, 59, 109) vorgetrieben werden.

23. Verfahren zur Vermischung der Ausgangskomponenten eines pastenförmigen Zementteigs, insbesondere eines pastenförmigen Mehrkomponenten-Polymethylmethacrylat-Knochenzementteigs, mit einem Lagerungs- und Mischsystem nach mindestens einem der vorangehenden Ansprüche, **gekennzeichnet durch** die folgenden nacheinander ablaufenden Schritte,
a) Entfernen des Kartuschenkopfs (9, 59, 109) von der ersten Kartusche (1, 51, 101) oder Öffnen der Öffnungen (22, 24, 72, 74) der ersten Kartusche (1, 51, 101) und der zweiten Kartusche (2, 52, 102),
b) Aufsetzen und Verbinden eines Austragsrohrs (26) mit der Vorderseite der ersten Kartusche (1, 51, 101),
c) Einsetzen der ersten Kartusche (1, 51, 101) in eine Auspressvorrichtung, die Auspressvorrichtung aufweisend einen axial vortreibbaren Stößel zum Vortreiben der Pressvorrichtung (7, 57, 80, 90, 107) im Inneren der ersten Kartusche (1, 51, 101) in Richtung des Austragsrohrs (26),
d) Auspressen der Ausgangskomponenten (3, 4, 53, 54) mit Hilfe der Auspressvorrichtung durch axiales Vortreiben des Stößels, wobei die Pressvorrichtung (7, 57, 80, 90, 107) mit dem Stößel in Richtung des Austragsrohrs (26) vorgetrieben wird, dabei der erste Austragskolben (5, 55, 105) von der Pressvorrichtung (7, 57, 80, 90, 107) in Richtung des Austragsrohrs (26) geschoben wird, die Klemmkante (8, 58, 82, 92, 108) der Pressvorrichtung (7, 57, 80, 90, 107) die Wand der zweiten Kartusche (2, 52, 102) zur Innenwand der ersten Kartusche (1, 51, 101) presst, dabei die verformte Wand der zweiten Kartusche (2, 52, 102) den zweiten Austragskolben (6, 56, 106) in der zweiten Kartusche (2, 52, 102) in Richtung des Austragsrohrs (26) schiebt, wodurch die Ausgangskomponenten (3, 4, 53, 54) des Zementteigs beider Kartuschen (1, 2, 51, 52, 101, 102) in das Austragsrohr (26) gedrückt werden, wobei die Ausgangskomponenten(3, 4, 53, 54) im Austragsrohr (26) zu dem pastenförmigen Zementteig gemischt werden und der gemischte Zementteig aus einer Austragsöffnung (36) des Austragsrohrs (26) ausfließt.

24. Verfahren nach Anspruch 23, **dadurch gekennzeichnet, dass** in Schritt a) die dritte Kartusche (61) nach einem der Ansprüche 20 oder 21 geöffnet wird, und bevorzugt auch die zumindest eine vierte Kartusche nach Anspruch 22 geöffnet wird, und in Schritt d) beim Vortreiben der Pressvorrichtung (7, 57, 80, 90, 107) mit dem Stößel in Richtung des Austragsrohrs (26) die Klemmkante (8, 58, 82, 92, 108) der Pressvorrichtung (7, 57, 80, 90, 107) die Wand der dritten Kartusche (61) zur Innenwand der ersten Kartusche (1, 51, 101) presst, dabei die verformte Wand der dritten Kartusche (61) den dritten Austragskolben (67) in der dritten Kartusche (61) in Richtung des Austragsrohrs (26) schiebt und bevorzugt beim Vortreiben der Pressvorrichtung (7, 57, 80, 90, 107) mit dem Stößel in Richtung des Austragsrohrs (26) die Klemmkante (8, 58, 82, 92, 108) der Pressvorrichtung (7, 57, 80, 90, 107) auch die jeweilige Wand der zumindest einen vierten Kartusche zur Innenwand der ersten Kartusche (1, 51, 101) presst, dabei die verformte Wand oder die verformten Wände der zumindest einen vierten Kartusche den jeweiligen vierten Austragskolben in der zumindest einen vierten Kartusche in Richtung des Austragsrohrs (26) schiebt.

25. Verfahren nach Anspruch 23 oder 24, **dadurch gekennzeichnet, dass** die Auspressvorrichtung manuell, mit Druckluft oder mit einem Motor angetrieben wird, wobei durch die manuelle Kraft, die Druckluft oder den Motor der Stößel in Richtung des Austragsrohrs (26) vorgetrieben wird.

## Claims

1. Storage and mixing system for pasty multicomponent polymethylmethacrylate bone cements, the storage and mixing system comprising
a first tubular cartridge (1, 51, 101) with a first cylindrical internal space, whereby a first starting component (3, 53) of a multicomponent bone cement is contained in the internal space;
a first dispensing plunger (5, 55, 105) that is arranged in the first internal space of the first cartridge (1,51, 101) such as to be axially mobile and that is provided for expelling the first starting component (3, 53) from the first cartridge (1, 51, 101) through an opening (22, 72) in the cartridge head (9, 59, 109) of the first cartridge (1, 51, 101) that is opposite from the first dispensing plunger (5, 55, 105);
a second tubular cartridge (2, 52, 102) that is arranged within the first tubular cartridge (1, 51, 101), whereby the external wall of the second cartridge (2, 52, 102) touches against the internal wall of the first cartridge (1, 51, 101) and is attached to the internal wall of the first cartridge (1, 51, 101), whereby the second cartridge (2, 52, 102) contains a second starting component (4, 54) of the multicomponent bone cement and has a second dispensing plunger (6, 56, 106) arranged in it, whereby the second dispensing plunger (6, 56, 106) can be used to expel the second starting component (4, 54) from the second cartridge (2, 52, 102) through an opposite opening (24, 74) in the second cartridge (2, 52, 102) in the region of the cartridge head (9, 59, 109) of the first cartridge (1, 51, 101);
whereby a pressing device (7, 57, 80, 90, 107) with a clamping edge (8, 58, 82, 92, 108) for compressing the second cartridge (2, 52, 102) that can be propelled axially in the internal space of the first cartridge (1, 51, 101) is arranged, as seen from the cartridge head (9, 59, 109), behind the first dispensing plunger (5, 55, 105) and the second dispensing plunger (6, 56, 106), whereby the pressing device (7, 57, 80, 90, 107) can be propelled appropriately in the direction of the cartridge head (9, 59, 109) such that the second cartridge (2, 52, 102) is being progressively compressed axially during the motion of the pressing device (7, 57, 80, 90, 107) such that, in the process, the first dispensing plunger (5, 55, 105) and the second dispensing plunger (6, 56, 106) are propelled in the direction of the cartridge head (9, 59, 109).

2. Storage and mixing system according to claim 1, **characterised in that** the external wall of the second cartridge (2, 52, 102) is attached to the internal wall of the first cartridge (1, 51, 101), in the front in the area of the cartridge head (9, 59, 109) and in the back behind the second dispensing plunger (6, 56, 106), whereby the external wall of the second cartridge (2, 52, 102) preferably is attached to the internal wall of the first cartridge (1, 51, 101) along the entire length of the second cartridge (2, 52, 102).

3. Storage and mixing system according to claim 1 or 2, **characterised in that** the openings (22, 24, 72, 74) a closed on the cartridge head (9, 59, 109) by means of a detachable closure (12, 14, 62, 64, 112, 114).

4. Storage and mixing system according to any one of the preceding claims, **characterised in that**
the first dispensing plunger (5, 55, 105) and the second dispensing plunger (6, 56, 106) are propelled parallel with respect to each other during the propulsion of the pressing device (7, 57, 80, 90, 107), preferably the first dispensing plunger (5, 55, 105) and the second dispensing plunger (6, 56, 106) run at the same level in the direction of the cartridge head (9, 59, 109).

5. Storage and mixing system according to any one of the preceding claims, **characterised in that**
an attachment means (16, 66, 116), in particular an external thread (16, 66, 116), is provided on the outside of the first cartridge (1, 51, 101) in the region of the cartridge head (9, 59, 109).

6. Storage and mixing system according to any one of the preceding claims, **characterised in that**
the storage and mixing system comprises a dispensing tube (26) with a static mixer (34) that can be attached to the first cartridge (1, 51, 101), preferably can be attached to the attachment means (16, 66, 116) on the first cartridge (1, 51, 101), whereby it is particularly preferred to provide on the dispensing tube (26) an internal thread (28) matching the external thread (16, 66, 116) on the first cartridge (1, 51, 101) and/or to provide elements of a bayonet closure and/or snap-in elements of a snap-in closure.

7. Storage and mixing system according to claim 6, **characterised in that** the ratio of the internal diameter of the first cartridge (1, 51, 101) and the internal diameter of the dispensing tube (26) is less than 5 to 2, whereby the ratio of the internal diameter of the first cartridge (1, 51, 101) and the internal diameter of the dispensing tube (26) preferably is less than or equal to 2 to 1, and particularly preferably the ratio of the internal diameter of the first cartridge (1, 51, 101) and the internal diameter of the dispensing tube (26) is 8 to 5.

8. Storage and mixing system according to any one of the preceding claims, **characterised in that**
the clamping edge (8, 58, 82, 92, 108) is inclined with respect to the longitudinal axis of the first cartridge (1, 51, 101), preferably is inclined at an angle of at least 40° perpendicular to the longitudinal axis in the direction of the internal wall of the first cartridge (1, 51, 101), particularly preferably is inclined at an angle between 40° and 80° perpendicular to the longitudinal axis in the direction of the internal wall of the first cartridge (1, 51, 101).

9. Storage and mixing system according to any one of the preceding claims, **characterised in that**
the clamping edge (8, 58, 82, 92, 108) squeezes the second cartridge (2, 52, 102) against the internal wall of the first cartridge (1, 51, 101) when the pressing device (7, 57, 80, 90, 107) is being propelled.

10. Storage and mixing system according to any one of the preceding claims, **characterised in that**
the clamping edge (8, 58, 82, 92, 108) covers at least 30% of the surface area of the cross-section of the second cartridge (2, 52, 102), preferably covers at least 60% of the surface area of the cross-section of the second cartridge (2, 52, 102).

11. Storage and mixing system according to any one of the preceding claims, **characterised in that**
a gap is provided between the pressing device (7, 57, 80, 90, 107) and the internal wall of the first cartridge (1, 51, 101) in the region of the second cartridge (2, 52, 102), whereby the gap is as wide as or wider than the thickness of the wall of the second cartridge (2, 52, 102).

12. Storage and mixing system according to any one of the preceding claims, **characterised in that**
the rear side of the pressing device (7, 57, 80, 90, 107) is designed as a support surface (84, 94) for a pestle of an extrusion device.

13. Storage and mixing system according to any one of the preceding claims, **characterised in that**
the diameter of the internal space of the first cartridge (1, 51, 101) is smaller than or equal to 25 mm, whereby the diameter of the internal space of the first cartridge (1, 51, 101) preferably is smaller than or equal to 20 mm, and/or the first cartridge (1, 51, 101) has an internal diameter of at most 25 mm and the second cartridge (2, 52, 102) has an internal diameter of at most 5 mm, preferably the first cartridge (1, 51, 101) has an internal diameter of at most 20 mm and the second cartridge to (2, 52, 102) has an internal diameter of at most 3 mm.

14. Storage and mixing system according to any one of the preceding claims, **characterised in that**
the clamping edge (8, 58, 82, 92, 108), by squeezing the second cartridge (2, 52, 102), presses the thus deformed wall of the second cartridge (2, 52, 102) against the underside of the second dispensing plunger (6, 56, 106) and thus pushes the second dispensing plunger (6, 56, 106) in the direction of the cartridge head (9, 59, 109) while the pressing device (7, 57, 80, 90, 107) is being propelled in the direction of the cartridge head (9, 59, 109).

15. Storage and mixing system according to any one of the preceding claims, **characterised in that**
the first cartridge (1, 51, 101), the second cartridge (2, 52, 102), the cartridge head (9, 59, 109), and the dispensing plungers (5, 6, 55, 56, 63, 105, 106) are made from plastic material, whereby polyethylene-co-vinylalcohol (EVOH), polybutylene-terephthalate (PBT), polyethylene-terephthalate (PET), and polymethacryl acid methylester-co-acrylonitrile are preferred as plastic materials.

16. Storage and mixing system according to any one of the preceding claims, **characterised in that**
the ratio of the volume of the first cartridge (1, 51, 101) and the volume of the second cartridge (2, 52, 102) is at least 95 to 5, preferably is at least 98 to 2.

17. Storage and mixing system according to any one of the preceding claims, **characterised in that**
the second cartridge (2, 52, 102) is squeezed appropriately, when the pressing device (7, 57, 80, 90, 107) is exposed to at least 0.5 kN acting in the direction of the cartridge head (9, 59, 109), such that the squeezed second cartridge (2, 52, 102) fits through a gap between the clamping edge (8, 58, 82, 92, 108) and the internal wall of the first cartridge (1, 51, 101).

18. Storage and mixing system according to any one of the preceding claims, **characterised in that**
the ratio of the thickness of the wall of the first cartridge (1, 51, 101) and the thickness of the wall of the second cartridge (2, 52, 102) is at least 11 to 10, and whereby the ratio of the thickness of the wall of the first cartridge (1, 51, 101) and the thickness of the wall of the second cartridge (2, 52, 102) is at least 2 to 1.

19. Storage and mixing system according to any one of the preceding claims, **characterised in that**
a part of the wall of the first cartridge (1, 51, 101) forms a part of the internal wall of the second cartridge (2, 52, 102), preferably over the entire length of the second cartridge (2, 52, 102) or over at least 80% of the entire length of the second cartridge (2, 52, 102), and/or a part of the wall of the first cartridge (1, 51, 101) limits a part of the second internal space of the second cartridge (2, 52, 102), preferably over the entire length of the second cartridge (2, 52, 102) or over at least 80% of the entire length of the second cartridge (2, 52, 102).

20. Storage and mixing system according to any one of the preceding claims, **characterised in that**
a third tubular cartridge (61) is arranged within the first tubular cartridge (1, 51, 101), whereby the external wall of the third cartridge (2, 52, 61) touches against the internal wall of the first cartridge (1, 51, 101) and is attached to the internal wall of the first cartridge (1, 51, 101, whereby the third cartridge (61) contains the second starting component (4, 54) or a third starting component (63) of the multicomponent bone cement and has a third dispensing plunger (67) arranged in it, whereby the third dispensing plunger (67) can be used to expel the second starting component (4, 54) for the third starting component (63) from the third cartridge (61) through an opposite opening (73) in the third cartridge (61) in the region of the cartridge head (9, 59, 109) of the first cartridge (1, 51, 101), whereby
the pressing device (7, 57, 80, 90, 107) is arranged, as seen from the cartridge head (9, 59, 109), behind the third dispensing plunger (67) and the pressing device (7, 57, 80, 90, 107) comprises a clamping edge (8, 58, 82, 92, 108) for compressing the third cartridge (61), whereby the pressing device (7, 57, 80, 90, 107) can be propelled can be propelled appropriately in the direction of the cartridge head (9, 59, 109) such that the third cartridge (61) is being progressively compressed axially during the motion of the pressing device (7, 57, 80, 90, 107) such that, in the process, the first dispensing plunger (5, 55, 105), the second dispensing plunger (6, 56, 106), and the third dispensing plunger (67) are propelled in the direction of the cartridge head (9, 59, 109).

21. Storage and mixing system according to claim 20, **characterised in that** the third cartridge (61) and/or the third dispensing plunger (67) have the same features as the second cartridge (2, 52, 102) and/or the second dispensing plunger (6, 56, 106) according to any one of the claims 2 to 19.

22. Storage in mixing system according to any one of the claims 20 or 21, **characterised in that**
at least one fourth tubular cartridge is arranged inside the first tubular cartridge (1, 51, 101), whereby the external wall of the at least one fourth cartridge touches against the internal wall of the first cartridge (1, 51, 101) and is attached to the internal wall of the first cartridge (1, 51, 101), whereby the at least one fourth cartridge contains the second, the third, a fourth and/or each at least one further starting component of the bone cement and the at least one fourth cartridge has a fourth dispensing plunger each arranged in it, whereby the second, the third, the fourth and/or the respective further starting component can be expelled from the at least one fourth cartridge, by means of the fourth dispensing plunger, through an opposite opening in the at least one fourth cartridge in the region of the cartridge head (9, 59, 109) of the first cartridge (1, 51, 101),
whereby the pressing device (7, 57, 80, 90, 107) is arranged behind the fourth dispensing plunger or plungers, as seen from the cartridge head (9, 59, 109, and the pressing device (7, 57, 80, 90, 107) comprises at least one clamping edge (8, 58, 82, 92, 108) for compressing the at least one fourth cartridge, whereby the pressing device (7, 57, 80, 90, 107) can be propelled appropriately in the direction of the cartridge head (9, 59, 109) such that the at least one fourth cartridge is progressively compressed axially while the pressing device (7, 57, 80, 90, 107) moves and thus the first dispensing plunger (5, 55, 105), the second dispensing plunger (6, 56, 106), the third dispensing plunger (67), and the fourth dispensing plunger or plungers are being propelled in the direction of the cartridge head (9, 59, 109).

23. Method for the mixing of the starting components of a pasty cement dough, in particular of a pasty multicomponent polymethylmethacrylate bone cement dough, using a storage and mixing system according to at least one of the preceding claims, **characterised by** the following steps proceeding in the given order,
a) Removing the cartridge head (9, 59, 109) from the first cartridge (1, 51, 101) or opening the openings (22, 24, 72, 74) of the first cartridge (1, 51, 101) and of the second cartridge (2, 52, 102);
b) attaching and connecting a dispensing tube (26) to the front side of the first cartridge (1, 51, 101);
c) inserting the first cartridge (1, 51, 101) into an extrusion device, whereby the extrusion device comprises a pestle that can be propelled axially for propelling the pressing device (7, 57, 80, 90, 107) on the interior of the first cartridge (1, 51, 101) in the direction of the dispensing tube (26);
d) extruding the starting components (3, 4, 53, 54) by means of the extrusion device by axially propelling the pestle, whereby the pressing device (7, 57, 80, 90, 107) is propelled in the direction of the dispensing tube (26) by the pestle, the first dispensing plunger (5, 55, 105) is pushed in the direction of the dispensing tube (26) by the pressing device (7, 57, 80, 90, 107), the clamping edge (8, 58, 82, 92, 108) of the pressing device presses the wall of the second cartridge(2, 52, 102) to the internal wall of the first cartridge (1, 51, 101), the deformed wall of the second cartridge (2, 52, 102) pushes the second dispensing plunger (6, 56, 106) in the second cartridge (2, 52, 102) in the direction of the dispensing tube (26), whereby the starting components (3, 4, 53, 54) of the cement dough of both cartridges (1, 2, 51, 52, 101, 102) are being pushed into the dispensing tube (26), whereby the starting components (3, 4, 53, 54) are mixed in the dispensing tube (26) to form the pasty cement dough and the mixed cement dough flows out from a dispensing opening (36) of the dispensing tube (26).

24. Method according to claim 23, **characterised in that**
the third cartridge (61) is opened in step a) according to any one of the claims 20 or 21, and preferably the at least one fourth cartridge is also opened according to claim 22, and during propulsion of the pressing device (7, 57, 80, 90, 107) with the pestle in the direction of the dispensing tube (26) in step d), the clamping edge (8, 58, 82, 92, 108) of the pressing device (7, 57, 80, 90, 107) presses the wall of the third cartridge (61) to the internal wall of the first cartridge (1, 51, 101), the deformed wall of the third cartridge (61) pushes the third dispensing plunger (67) in the third cartridge (61) in the direction of the dispensing tube (26) and, preferably, during propulsion of the pressing device (7, 57, 80, 90, 107) with a pestle in the direction of the dispensing tube (26), the clamping edge (8, 58, 82, 92, 108) of the pressing device (7, 57, 80, 90, 107) also presses the respective wall of the at least one fourth cartridge to the internal wall of the first cartridge (1, 51, 101), the deformed wall or the deformed walls of the at least one fourth cartridge push(es) the respective fourth dispensing plunger in the at least one fourth cartridge in the direction of the dispensing tube (26).

25. Method according to claim 23 or 24, **characterised in that** the extrusion device is driven manually, by compressed air or by a motor, whereby the manual force, the compressed air or the motor propels the pestle in the direction of the dispensing tube (26).

## Revendications

1. Système de stockage et de mélange de ciments osseux à plusieurs composants de polyméthyl méthacrylate de forme pâteuse, le système de stockage et de mélange présentant
une première cartouche (1, 51, 101) en forme de tube avec un premier espace intérieur cylindrique, où un premier composant de départ (3, 53) d'un ciment osseux à plusieurs composants est contenu dans l'espace intérieur,
un premier piston de décharge (5, 55, 105) qui est disposé mobile axialement dans le premier espace intérieur de la première cartouche (1, 51, 101) et qui est prévu pour l'expulsion du premier composant de départ (3, 53) hors de la première cartouche (1, 51, 101) par un orifice (22, 72) situé en face du premier piston de décharge (5, 55, 105) dans une tête de cartouche (9, 59, 109) de la première cartouche (1, 51, 101),
une deuxième cartouche (2, 52, 102) en forme de tube qui est disposée à l'intérieur de la première cartouche (1, 51, 101) en forme de tube, où la deuxième cartouche (2, 52, 102) se plaque avec sa paroi extérieure contre la paroi intérieure de la première cartouche (1, 51, 101) et est fixée sur la paroi intérieure de la première cartouche (1, 51, 101), où un deuxième composant de départ (4, 54) du ciment osseux à plusieurs composants est contenu dans la deuxième cartouche (2, 52, 102) et où un deuxième piston de décharge (6, 56, 106) est disposé, où le deuxième composant de départ (4, 54) doit être expulsé avec le deuxième piston de décharge (6, 56, 106) hors de la deuxième cartouche (2, 52, 102) par un orifice (24, 74) se situant en face dans la deuxième cartouche (2, 52, 102), dans la région de la tête de cartouche (9, 59, 109) de la première cartouche (1, 51, 101),
où, vu à partir de la tête de cartouche (9, 59, 109), derrière le premier piston de décharge (5, 55, 105) et le deuxième piston de décharge (6, 56, 106), un dispositif de pressage (7, 57, 80, 90, 107) pouvant être entraîné axialement dans l'espace intérieur de la première cartouche (1, 51, 101) est disposé avec un bord de serrage (8, 58, 82, 92, 108) pour la compression de la deuxième cartouche (2, 52, 102), où le dispositif de pressage (7, 57, 80, 90, 107) peut être entraîné en direction de la tête de cartouche (9, 59, 109) de telle manière que, pendant le déplacement du dispositif de pressage (7, 57, 80, 90, 107), la deuxième cartouche (2, 52, 102) est compressée axialement de manière continue et ainsi le premier piston de décharge (5, 55, 105) et le deuxième piston de décharge (6, 56, 106) sont avancés en direction de la tête de cartouche (9, 59, 109).

2. Système de stockage et de mélange selon la revendication 1, **caractérisé en ce que** la deuxième cartouche (2, 52, 102) est fixée avec sa paroi extérieure avec la paroi intérieure de la première cartouche (1, 51, 101) à l'avant dans la région de la tête de cartouche (9, 59, 109) et à l'arrière, derrière le deuxième piston de décharge (6, 56, 106), où, de préférence, la deuxième cartouche (2, 52, 102) est fixée avec sa paroi extérieure avec la paroi intérieure de la première cartouche (1, 51, 101) le long de la longueur totale de la deuxième cartouche (2, 52, 102).

3. Système de stockage et de mélange selon la revendication 1 ou la revendication 2, **caractérisé en ce que**
les orifices (22, 24, 72, 74) sont fermés avec un système de fermeture (12, 14, 62, 64, 112, 114) amovible sur la tête de cartouche (9, 59, 109).

4. Système de stockage et de mélange selon l'une des revendications précédentes, **caractérisé en ce que**,
lors de l'avancée du dispositif de pressage (7, 57, 80, 90, 107), le premier piston de décharge (5, 55, 105) et le deuxième piston de décharge (6, 56, 106) sont avancés parallèlement l'un par rapport à l'autre, de préférence, le premier piston de décharge (5, 55, 105) et le deuxième piston de décharge (6, 56, 106) se déplacent à la même hauteur en direction de la tête de cartouche (9, 59, 109).

5. Système de stockage et de mélange selon l'une des revendications précédentes, **caractérisé en ce que**,
dans la région de la tête de cartouche (9, 59, 109), un système de fixation (16, 66, 116), notamment, un filetage extérieur (16, 66, 116) est prévu à l'extérieur sur la première cartouche (1, 51, 101).

6. Système de stockage et de mélange selon l'une des revendications précédentes, **caractérisé en ce que**
le système de stockage et de mélange présente un tuyau d'évacuation (26) avec un mélangeur statique (34) qui doit être fixé sur la première cartouche (1, 51, 101), de préférence, doit être fixé sur le système de fixation (16, 66, 116) au niveau de la première cartouche (1, 51, 101), où, de manière particulièrement préférée, un filetage intérieur (28) correspondant au filetage extérieur (16, 66, 116) est prévu sur la première cartouche (1, 51, 101), et/ou des éléments d'un système de fermeture à baïonnette et/ou des éléments de verrouillage d'un système de fermeture par encliquetage.

7. Système de stockage et de mélange selon la revendication 6, **caractérisé en ce que** le rapport entre le diamètre intérieur de la première cartouche (1, 51, 101) sur le diamètre intérieur du tuyau d'évacuation (26) est inférieur à 5 sur 2, où, de préférence, le rapport entre le diamètre intérieur de la première cartouche (1, 51, 101) sur le diamètre intérieur du tuyau d'évacuation (26) est de préférence inférieur ou égal à 2 sur 1 et de manière particulièrement préférée, le rapport du diamètre intérieur de la première cartouche (1, 51, 101) sur le diamètre intérieur du tuyau d'évacuation (26) est de 8 sur 5.

8. Système de stockage et de mélange selon l'une des revendications précédentes, **caractérisé en ce que**
le bord de serrage (8, 58, 82, 92, 108) est incliné contre l'axe longitudinal de la première cartouche (1, 51, 101), de préférence, est incliné avec un angle d'au moins 40 ° perpendiculairement par rapport à l'axe longitudinal en direction de la paroi intérieure de la première cartouche (1, 51, 101), de manière particulièrement préférée, est incliné avec un angle entre 40 ° e 80 ° perpendiculairement par rapport à l'axe longitudinal en direction de la paroi intérieure de la première cartouche (1, 51, 101).

9. Système de stockage et de mélange selon l'une des revendications précédentes, **caractérisé en ce que**
le bord de serrage (8, 58, 82, 92, 108) écrase la deuxième cartouche (2, 52, 102) contre la paroi intérieure de la première cartouche (1, 51, 101) lors de l'avancée du dispositif de pressage (7, 57, 80, 90, 107).

10. Système de stockage et de mélange selon l'une des revendications précédentes, **caractérisé en ce que**
le bord de serrage (8, 58, 82, 92, 108) recouvre au moins 30 pourcent de la surface de la section transversale de la deuxième cartouche (2, 52, 102), de préférence, recouvre au moins 60 % de la surface de la section transversale de la deuxième cartouche (2, 52, 102).

11. Système de stockage et de mélange selon l'une des revendications précédentes, **caractérisé en ce**
**qu'**une fente est prévue entre le dispositif de pressage (7, 57, 80, 90, 107) et la paroi intérieure de la première cartouche (1, 51, 101) dans la région de la deuxième cartouche (2, 52, 102), fente qui est exactement aussi large ou plus large que l'épaisseur de la paroi de la deuxième cartouche (2, 52, 102).

12. Système de stockage et de mélange selon l'une des revendications précédentes, **caractérisé en ce que**
la face arrière du dispositif de pressage (7, 57, 80, 90, 107) est conçue sous forme d'une surface de support (84, 94) pour un poussoir d'un dispositif d'expulsion.

13. Système de stockage et de mélange selon l'une des revendications précédentes, **caractérisé en ce que**
le diamètre de l'espace intérieur de la première cartouche (1, 51, 101) est inférieur ou égal à 25 mm, où, de préférence, le diamètre de l'espace intérieur de la première cartouche (1, 51, 101) est inférieur ou égal à 20 mm, et/ou la première cartouche (1, 51, 101) a un diamètre intérieur d'au maximum 25 mm et la deuxième cartouche (2, 52,102) a un diamètre intérieur d'au maximum 5 mm, de préférence, la première cartouche (1, 51, 101) a un diamètre intérieur d'au maximum 20 mm et la deuxième cartouche (2, 52, 102) a un diamètre intérieur d'au maximum 3 mm.

14. Système de stockage et de mélange selon l'une des revendications précédentes, **caractérisé en ce que**,
lors de l'avancée du dispositif de serrage (7, 57, 80, 90, 107) en direction de la tête de cartouche (9, 59, 109), le bord de serrage (8, 58, 82, 92, 108) presse la paroi ainsi déformée de la deuxième cartouche (2, 52, 102) contre la face inférieure du deuxième piston de décharge (6, 56, 106) par un écrasement de la deuxième cartouche (2, 52, 102), et ainsi déplace le deuxième piston de décharge (6, 56, 106) en direction de la tête de cartouche (9, 59, 109).

15. Système de stockage et de mélange selon l'une des revendications précédentes, **caractérisé en ce que**
la première cartouche (1, 51, 101), la deuxième cartouche (2, 52, 102), la tête de cartouche (9, 59, 109) et les pistons de décharge (5, 6, 55, 56, 63, 105, 106) sont fabriqués en matière plastique, où, en tant que matières plastiques, on préfère du copolymère polyéthylène-alcool vinylique (EVOH), du polybutylène téréphtalate (PBT), du polyéthylène téréphtalate (PET) et du copolymère polyester méthyl méthacrylate-co-acrylonitrile.

16. Système de stockage et de mélange selon l'une des revendications précédentes, **caractérisé en ce que**
le rapport du volume de la première cartouche (1, 51, 101) sur le volume de la deuxième cartouche (2, 52, 102) est d'au moins 95 sur 5, de préférence, d'au moins 98 sur 2.

17. Système de stockage et de mélange selon l'une des revendications précédentes, **caractérisé en ce que**
la deuxième cartouche (2, 52, 102) est écrasée en direction de la tête de cartouche (9, 59, 109) lors d'une sollicitation du dispositif de pressage (7, 57, 80, 90, 107) d'au moins 0,5 kN de sorte que la deuxième cartouche (2, 52, 102) écrasée par la fente s'adapte entre le bord de serrage (8, 58, 82, 92, 108) et la paroi intérieure de la première cartouche (1, 51, 101).

18. Système de stockage et de mélange selon l'une des revendications précédentes, **caractérisé en ce que**
le rapport de l'épaisseur de la paroi de la première cartouche (1, 51, 101) sur l'épaisseur de la paroi de la deuxième cartouche (2, 52, 102) est d'au moins 11 sur 10, et où, de préférence, le rapport de l'épaisseur de la paroi de la première cartouche (1, 51, 101) sur l'épaisseur de la paroi de la deuxième cartouche (2, 52, 102) est d'au moins 2 sur 1.

19. Système de stockage et de mélange selon l'une des revendications précédentes, **caractérisé en ce**
**qu'**une partie de la paroi de la première cartouche (1, 51, 101) forme une partie de la paroi intérieure de la deuxième cartouche (2, 52, 102), de préférence, sur la longueur totale de la deuxième cartouche (2, 52, 102) ou sur au moins 80 % de la longueur totale de la deuxième cartouche (2, 52, 102), et/ou qu'une partie de la paroi de la première cartouche (1, 51, 101) délimite une partie du deuxième espace intérieur de la deuxième cartouche (2, 52, 102), de préférence, sur la longueur totale de la deuxième cartouche (2, 52, 102) ou sur au moins 80 % de la longueur totale de la deuxième cartouche (2, 52, 102).

20. Système de stockage et de mélange selon l'une des revendications précédentes, **caractérisé en ce**
**qu'**une troisième cartouche (61) en forme de tube est disposée à l'intérieur de la première cartouche (1, 51, 101) en forme de tube, où la troisième cartouche (61) se plaque avec sa paroi extérieure contre la paroi intérieure de la première cartouche (1, 51, 101) et est fixée sur la paroi intérieure de la première cartouche (1, 51, 101), où le deuxième composant de départ (4, 54) ou un troisième composant de départ (63) du ciment osseux à plusieurs composants est contenu dans la troisième cartouche (61) et où un troisième piston de décharge (67) est disposé, où le deuxième composant de départ (4, 54) ou le troisième composant de départ (63) peut être expulsé avec le troisième piston de décharge (67) hors de la troisième cartouche (61) par un orifice (73) se situant en face dans la troisième cartouche (61) dans la région de la tête de cartouche (9, 59, 109) de la première cartouche (1, 51, 101), où où, vu à partir de la tête de cartouche (9, 59, 109), le dispositif de pressage (7, 57, 80, 90, 107) est disposé derrière le troisième piston de décharge (67) et le dispositif de décharge (7, 57, 80, 90, 107) présente un bord de serrage (8, 58, 82, 92, 108) pour la compression de la troisième cartouche (61), où le dispositif de pressage (7, 57, 80, 90, 107) peut être entraîné en direction de la tête de cartouche (9, 59, 109) de telle manière que, pendant le déplacement du dispositif de pressage (7, 57, 80, 90, 107), la troisième cartouche (61) est compressée axialement de manière continue et ainsi le premier piston de décharge (5, 55, 105), le deuxième piston de décharge (6, 56, 106) et le troisième piston de décharge (67) sont avancés en direction de la tête de cartouche (9, 59, 109).

21. Système de stockage et de mélange selon la revendication 20, **caractérisé en ce que** la troisième cartouche (61) et/ou le troisième piston de décharge (67) présentent les mêmes caractéristiques que la deuxième cartouche (2, 52, 102) et/ou le deuxième piston de décharge (6, 56, 106) selon l'une des revendications 2 à 19.

22. Système de stockage et de mélange selon l'une des revendications 20 ou 21, **caractérisé en ce**
**qu'**au moins une quatrième cartouche en forme de tube est disposée à l'intérieur de la première cartouche (1, 51, 101) en forme de tube, où l'au moins une quatrième cartouche se plaque avec sa paroi extérieure contre la paroi intérieure de la première cartouche (1, 51, 101) et est fixée sur la paroi intérieure de la première cartouche (1, 51, 101), où le deuxième, le troisième, un quatrième et/ou respectivement au moins un autre composant de départ du ciment osseux est contenu dans l'au moins une quatrième cartouche et où respectivement un quatrième piston de décharge est disposé dans l'au moins une quatrième cartouche, où le deuxième, le troisième, le quatrième et/ou l'autre composant de départ respectif peut être expulsé avec le quatrième piston de décharge hors de l'au moins une quatrième cartouche par un orifice se situant en face dans l'au moins une quatrième cartouche dans la région de la tête de cartouche (9, 59, 109) de la première cartouche (1, 51, 101), où,
vu à partir de la tête de cartouche (9, 59, 109), le dispositif de pressage (7, 57, 80, 90, 107) est disposé derrière le quatrième piston ou les quatrièmes pistons de décharge et le dispositif de décharge (7, 57, 80, 90, 107) présente au moins un bord de serrage (8, 58, 82, 92, 108) pour la compression de l'au moins une quatrième cartouche, où le dispositif de pressage (7, 57, 80, 90, 107) peut être entraîné en direction de la tête de cartouche (9, 59, 109) de telle manière que, pendant le déplacement du dispositif de pressage (7, 57, 80, 90, 107), l'au moins une quatrième cartouche est compressée axialement de manière continue et ainsi le premier piston de décharge (5, 55, 105), le deuxième piston de décharge (6, 56, 106) et le troisième piston de décharge (67) et le quatrième ou les quatrièmes pistons de décharge sont avancés en direction de la tête de cartouche (9, 59, 109).

23. Procédé de mélange des composants de départ d'une pâte de ciment de forme pâteuse, notamment d'une pâte de ciment osseux de polyméthyl méthacrylate à plusieurs composants de forme pâteuse, avec un système de stockage et de mélange selon au moins l'une des revendications précédentes, **caractérisé par** les étapes suivantes se déroulant les unes à la suite des autres,
a) le retrait de la tête de cartouche (9, 59, 109) de la première cartouche (1, 51, 101) ou l'ouverture des orifices (22, 4, 72, 74) de la première cartouche (1, 51, 101) et de la deuxième cartouche (2, 52, 102),
b) la mise en place et la connexion d'un tuyau d'évacuation (26) avec la face avant de la première cartouche (1, 51, 101),
c) l'insertion de la première cartouche (1, 51, 101) dans un dispositif d'expulsion par pressage, le dispositif d'expulsion par pressage présentant un poussoir pouvant être entraîné axialement pour l'avancée du dispositif de pressage (7, 57, 80, 90, 107) à l'intérieur de la première cartouche (1, 51, 101) en direction du tuyau d'évacuation (26),
d) d'expulsion des composants de départ (3, 4, 53, 54) à l'aide du dispositif d'expulsion par pressage par une avancée axiale du poussoir, où le dispositif de pressage (7, 57, 80, 90, 107) est déplacé vers l'avant avec le poussoir en direction du tuyau d'évacuation (26), où, ce faisant, le premier piston de décharge (5, 55, 105) est poussé par le dispositif de pressage (7, 57, 80, 90, 107) en direction du tuyau d'évacuation (26), le bord de serrage (8, 58, 82, 92, 108) du dispositif de pressage (7, 57, 80, 90, 107) pousse la paroi de la deuxième cartouche (2, 52, 102) vers la paroi intérieure de la première cartouche (1, 51, 101), ainsi la paroi déformée de la deuxième cartouche (2, 52, 102) pousse le deuxième piston de décharge (6, 56, 106) dans la deuxième cartouche (2, 52, 102) en direction du tuyau d'évacuation (26), ce par quoi, les composants de départ (3, 4, 53, 54) de la pâte de ciment des deux cartouches (1, 2, 51, 52, 101, 102) sont pressés dans le tuyau d'évacuation (26), où les composants de départ (3, 4, 53, 54) sont mélangés pour former la pâte de ciment de forme pâteuse et la pâte de ciment mélangée s'écoule hors d'un orifice d'évacuation (36) du tuyau d'évacuation (26).

24. Procédé selon la revendication 23, **caractérisé en ce que**
dans l'étape a), la troisième cartouche (61) selon l'une des revendications 20 ou 21 est ouverte, et de préférence, également l'au moins une quatrième cartouche selon la revendication 22 est ouverte, et dans l'étape d), lors de l'avancée du dispositif de pressage (7, 57, 80, 90, 107) avec le poussoir en direction du tuyau d'évacuation (26), le bord de serrage (8, 58, 92, 92, 108) du dispositif de pressage (7, 57, 80, 90, 107) presse la paroi de la troisième cartouche (61) vers la paroi intérieure de la première cartouche (1, 51, 101), ce faisant, la paroi déformée de la troisième cartouche (61) déplace le troisième piston de décharge (67) dans la troisième cartouche (61) en direction du tuyau d'évacuation (26) et de préférence, lors de l'avancée du dispositif de pressage (7, 57, 80, 90, 107) avec le poussoir en direction du tuyau d'évacuation (26), le bord de serrage (8, 58, 82, 92, 108) du dispositif de pressage (7, 57, 80, 90, 107) presse également la paroi respective de l'au moins une quatrième cartouche vers la paroi intérieure de la première cartouche (1, 51, 101), ainsi la paroi déformée ou les parois déformées de l'au moins une quatrième cartouche déplacent le quatrième piston de décharge respectif dans l'au moins une quatrième cartouche en direction du tuyau d'évacuation (26).

25. Procédé selon la revendication 23 ou la revendication 24, **caractérisé en ce que** le dispositif d'expulsion par pressage est entraîné manuellement, avec de l'air comprimé ou avec un moteur, où le poussoir est entraîné en direction du tuyau d'évacuation (26) par la force manuelle, l'air comprimé ou le moteur.
